# EUROPEAN PATENT APPLICATION

(11) **EP 4 844 014 A1**
(43) Date of publication of application: **25.11.2026**
(21) Application number: 25178568.9
(22) Date of filing: 23.05.2025
(51) Int. Cl.: C08G 73/02, A61K 45/06, A61K 47/59, C08G 83/00, C08L 79/02, C12N 15/88

(54) **MODIFIED POLYMERS**

(71) Applicant: Universität Leipzig, 04109 Leipzig (DE)
(72) Inventor: AIGNER, Achim, 04109 Leipzig (DE); MATTHÉ, Tanja, 04109 Leipzig (DE); EWE, Alexander, 04109 Leipzig (DE)
(74) Representative: Vossius & Partner Patentanwälte Rechtsanwälte mbB

(57) **Abstract**

The present invention provides for a polypropylenimine-dendrimer (PPI) comprising one or more N-Boc-tyrosine residue(s) and to a polyethylenimine (PEI) comprising one or more N-Boc-tyrosine residue(s). The herein provided PPI and PEI may further comprise one or more tyrosine residue(s). Further herein provided are compositions and nanoparticles comprising the herein provided PPI and/or PEI. Furthermore, the present invention provides for methods and uses employing said PPI, PEI, compositions, and/or nanoparticles.

## Description

The present invention provides for a polypropylenimine-dendrimer (PPI) comprising one or more N-Boc-tyrosine residue(s) and for a polyethylenimine (PEI) comprising one or more N-Boc-tyrosine residue(s). The herein provided PPI and PEI may further comprise one or more tyrosine residue(s). Further herein provided are compositions and nanoparticles comprising the herein provided PPI and/or PEI. Furthermore, the present invention provides for methods and uses employing said PPI, PEI, compositions, and/or nanoparticles. Further herein provided are methods for the production of the herein provided PPIs and/or PEIs, compositions, and nanoparticles.

Efficient delivery of nucleic acids in cells both *in vitro* and *in vivo* is relevant in the context of, *e.g.,* cancer therapeutics, vaccines, treatment of metabolic diseases, genetic disorders, neurological diseases, in particular in therapeutic approaches. Vehicles/carriers for nucleic acid delivery include, *inter alia,* lipid nanoparticles (LNPs). Alternatively, various polymer-based carriers have been developed that show other physicochemical and biological properties as compared to LNPs. Such polymers include polyethylenimines (PEI) and polypropylenimines, that can form linear or branched PEIs and polypropylenimine-dendrimers (PPI), respectively, all of which are now commercially available. Both PEIs and PPIs, have been previously formulated into nanoparticles comprising nucleic acids and employed in the transfection of cells with said nucleic acids.

Previously, it was demonstrated that modifications of PPIs and PEIs with tyrosine residues increases the transfection efficiency of nucleic acids when formulated with such PPIs or PEIs (see DE 102020 114 183 A1, WO 2009/074970 A1, WO 2011/120953 A1, and [1] to [7], all of which are herein incorporated by reference in their entirety). In particular, the transfection efficiency of small nucleic acids (particularly small RNA molecules such as small interfering RNAs, microRNAs, microRNA-mimics, and the like) could be increased by such tyrosine-modifications. Resultingly, tyrosine-modified PPIs and PEIs (or nanoparticles/compositions comprising the same) were particularly useful in *in vitro* and *in vivo* transfection of small RNAs for gene knockdowns.

However, there is still a need in the art to further improve PPIs, PEIs and compositions or nanoparticles comprising the same, in particular there is still a need to further enhance transfection efficiencies thereof, in particular regarding the transfection of larger RNAs like mRNAs, sgRNAs or others.

This technical problem is solved by the invention as described herein and as characterized in the appended claims.

In particular, this technical problem is solved by the provision of a polypropylenimine-dendrimer (PPI) comprising one or more N-Boc-tyrosine residue(s) and/or by the provision of a polyethylenimine (PEI) comprising one or more N-Boc-tyrosine residue(s) (collectively herein referred to as 'the herein provided compounds').

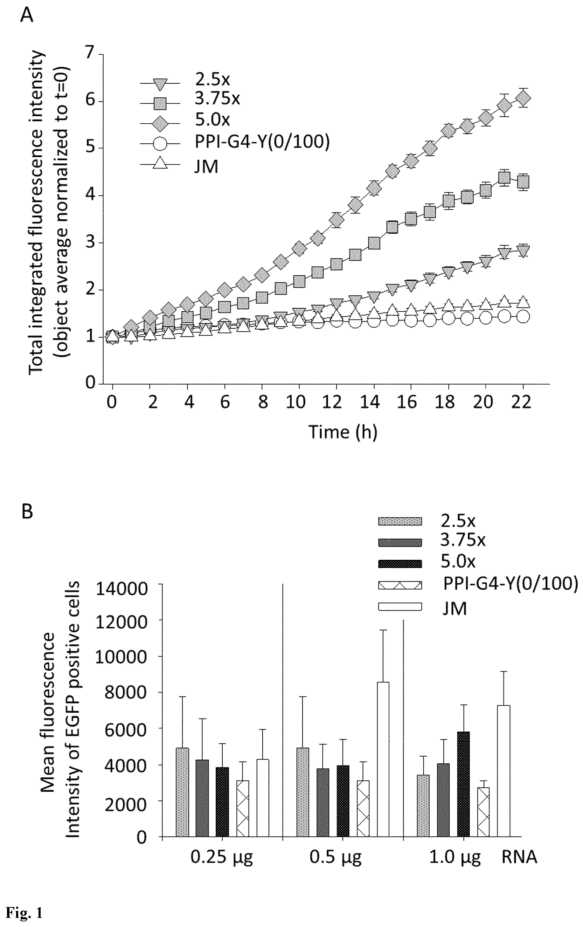

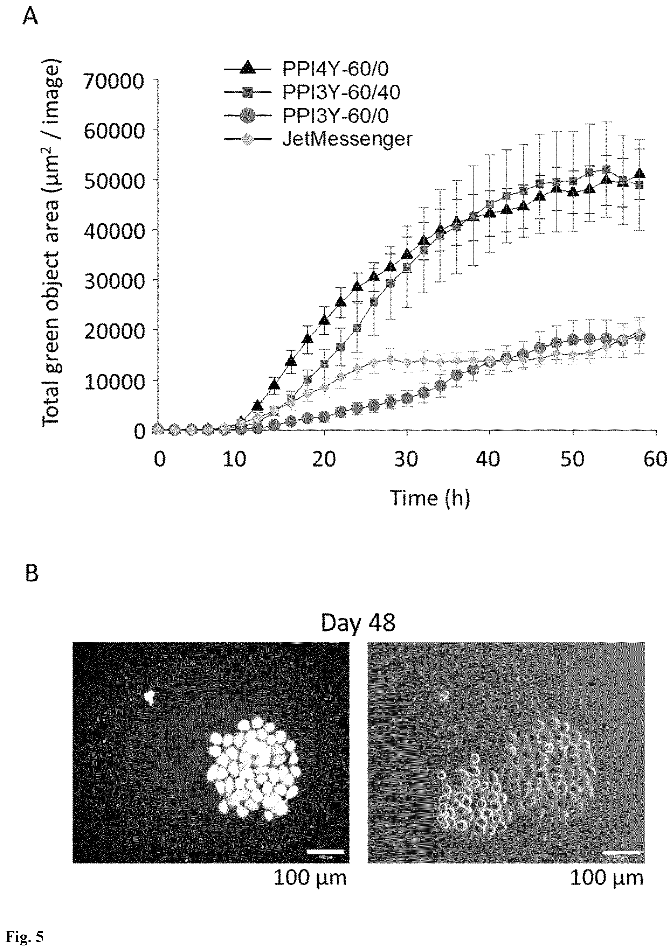

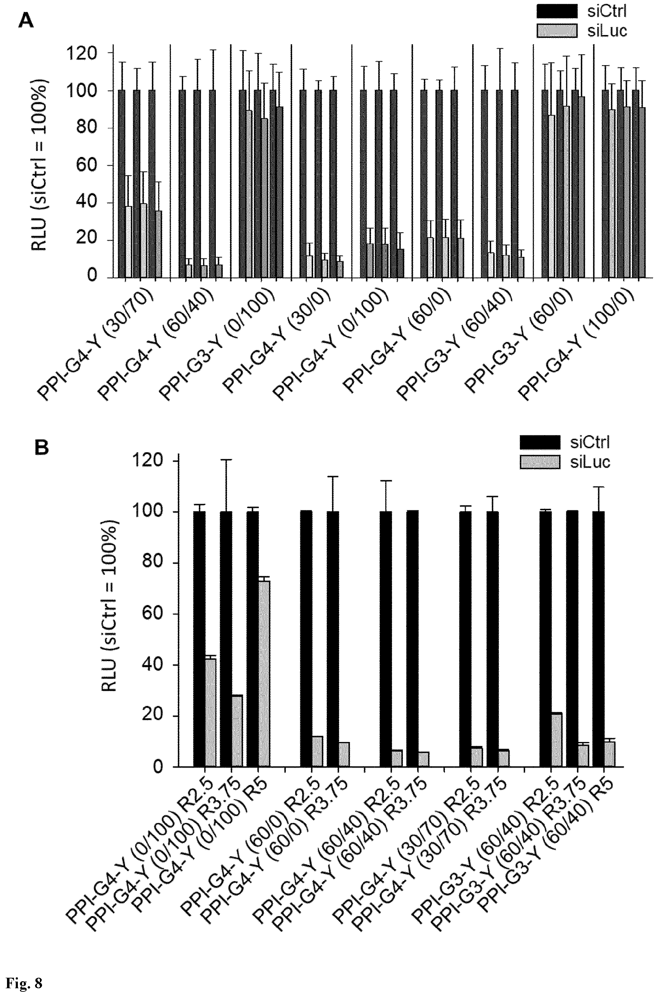

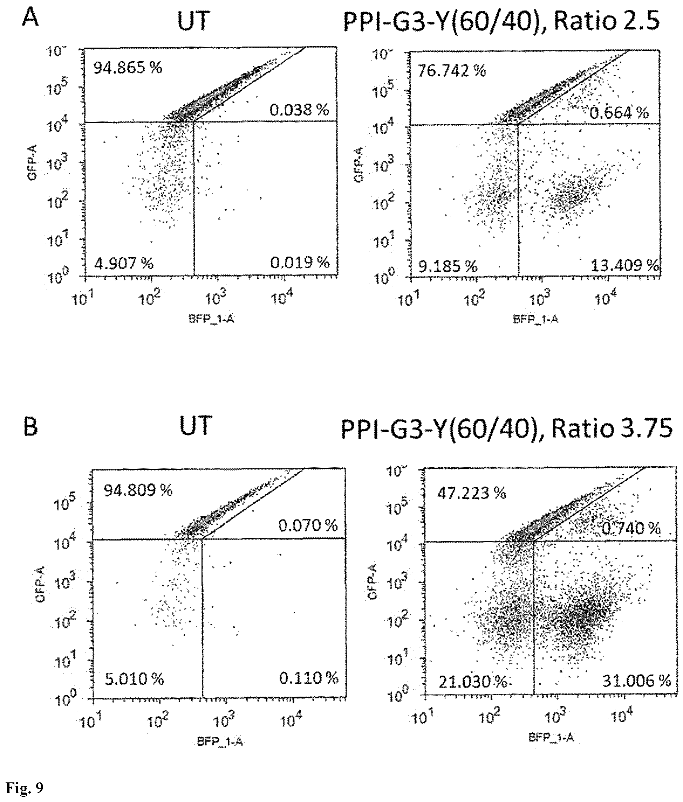

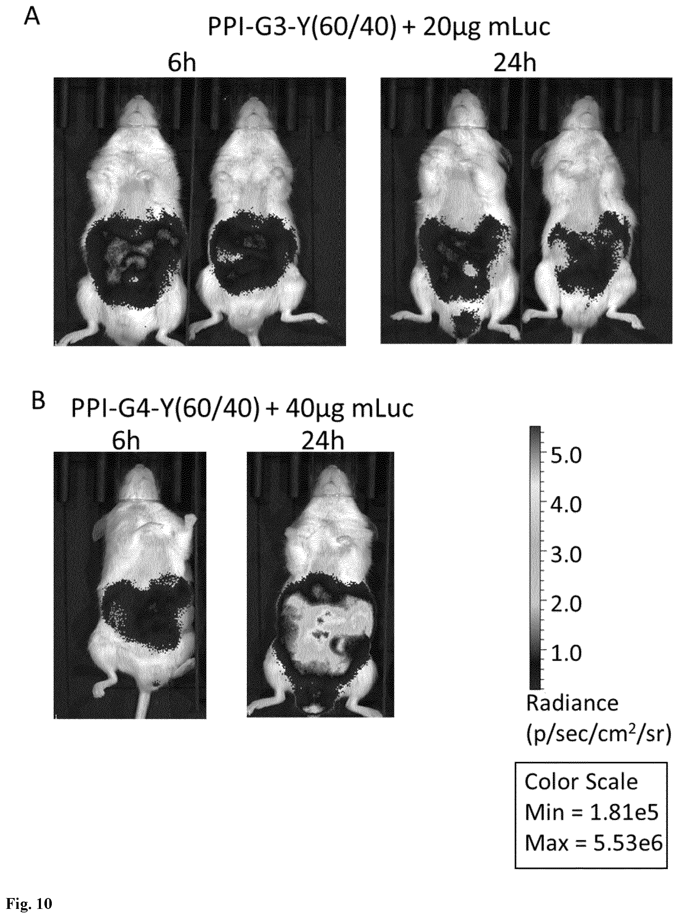

The advantageous and surprising technical effects of the herein provided compounds are evidenced by the enclosed illustrative and non-limiting examples and figures. The examples and figures, *inter alia,* illustrate that the herein provided compounds (or compositions or nanoparticles comprising the same):
- surprisingly show enhanced *in vitro* transfection efficiencies for RNAs, including messenger RNA (mRNA; see, *e.g.,* **Fig. 1**) and small interfering RNA (siRNA; see, *e.g.,* **Fig. 8**), as compared to state-of-the art PPIs or PEIs that comprise only tyrosine-modifications *(i.e.,* that do not comprise N-Boc-tyrosine residues);
- surprisingly show enhanced *in vitro* transfection efficiencies for mRNA as compared to a gold-standard mRNA-transfection reagent *(i.e.,* as compared to jetMessenger^{®} mRNA transfection reagent (Polyplus^{®} Sartorius); *c.f,* WO 2020 089342 A1, which is incorporated herein by reference in its entirety; see, *e.g.,* **Fig. 1**);
- could be employed in the delivery of single-guide RNAs (sgRNAs) and Cas-encoding mRNA resulting in base-editing events (see, **Fig. 9**);
- are surprisingly suitable for the transfection of larger nucleic acids (as compared to, *e.g.,* siRNAs as employed in the state of the art), in particular, the present inventors could, *inter alia,* employ the herein provided compounds in the transfection of large DNA molecules *(i.e.,* plasmids and minicircle DNA) and large RNA molecules *(i.e.,* self-amplifying mRNA (sa-mRNA); see *e.g.,* **Fig. 5**); and
- have been successfully employed in the delivery of multiple cargos/nucleic acids (see, *e.g.,* **Fig. 9**); and
- are suitable for and efficient in the *in vivo* transfection of nucleic acids (see, **Fig. 10**), and
- show high biocompatibility in vitro and in vivo.

None of the above described technical effects could have been foreseen. In fact, it could not have been foreseen that the herein provided compounds were suitable for transfections at all, as the present inventors accidentally conceived the present invention when attempting to produce the above-mentioned state of the art PPIs comprising tyrosine modifications. This is further illustrated in **Example 2,** herein below.

Accordingly, the present invention provides for a polypropylenimine (such as a polypropylenimine-dendrimer or a linear polypropylenimine) comprising one or more N-Boc-tyrosine residue(s), in particular a polypropylenimine-dendrimer (PPI) comprising one or more N-Boc-tyrosine residue(s), and for a polyethylenimine (PEI) comprising one or more N-Boc-tyrosine residue(s), optionally further comprising one or more tyrosine residue(s).

As indicated above, the herein provided compounds are particularly advantageous for transfections of *e.g.,* diverse DNAs and RNAs *in vivo* and *in vitro.* Accordingly, the herein provided PPIs and PEIs (or compositions or nanoparticles comprising the same) are particularly suitable for the transfection of DNA and/or RNA. The herein provided PPIs or PEIs (each comprising one or more N-Boc-tyrosine residue(s)) preferably comprise higher transfection efficiency when transfecting a cell with a polynucleotide (such as, DNA or RNA) as compared to a reference PPI or a reference PEI (e.g. each not comprising an N-Boc-tyrosine residue or each comprising one or more tyrosine residues as sole modification at the one or more (all) terminal amino group(s)), respectively, or as compared to a conventional mRNA-transfection reagent (*e.g*. as compared to jetMessenger^{®} mRNA transfection reagent), optionally, wherein transfection efficiency is assessed by quantifying expression of reporter gene encoded by said polynucleotide.

Illustrative and non-limiting examples of the herein provided PPIs are shown in **Formulas I** and **II**, showing PPIs of the third and fourth generations, respectively.

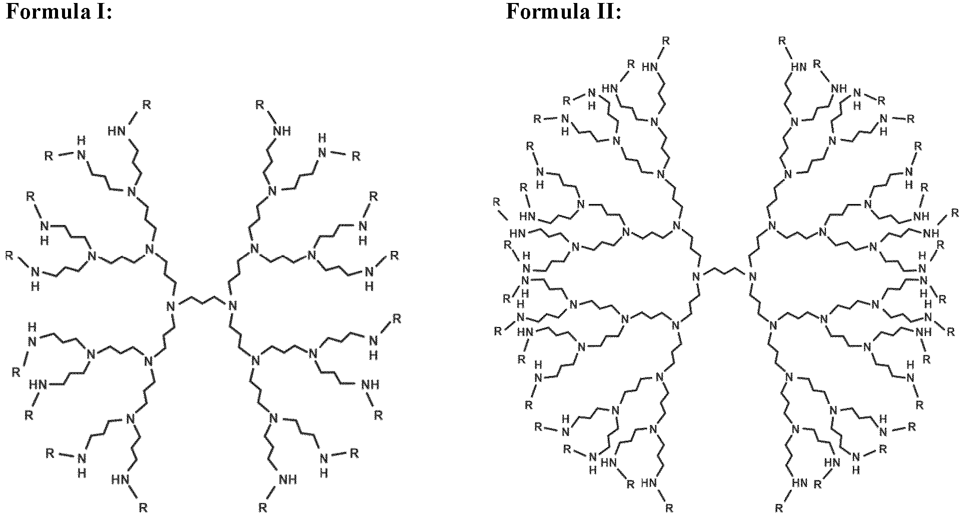

Illustrative and non-limiting examples of the herein provided PEIs are shown in **Formulas III** and **IV,** showing branched and linear PEIs, respectively.

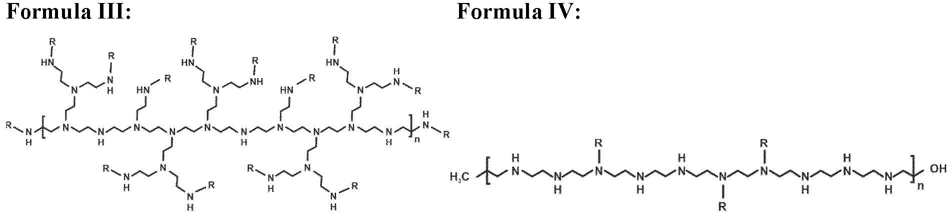

In any of **Formulas I** to **IV,** the indicated 'R's are independently from another selected from N-Boc-tyrosine, tyrosine, and hydrogen, with at least one 'R' being N-Boc-tyrosine.

The present invention is further characterized by the following items:
1. A composition comprising a polypropylenimine-dendrimer (PPI) or a polyethylenimine (PEI), wherein said PPI or said PEI comprises one or more N-Boc-tyrosine residue(s), and optionally wherein the composition further comprises one or more agent(s), and wherein said PEI is a linear PEI or a branched PEI.
2. The composition according to item 1, wherein said PPI or said PEI forms a nanoparticle and/or is formulated as a nanoparticle with said one or more agent(s).
3. The composition according to item 1 or 2, wherein said one or more agent(s) is/are one or more polynucleotide molecule(s), one or more polypeptide(s), and/or one or more small molecule(s), preferably one or more polynucleotide molecule(s).
4. The composition according to any one of item 1 to 3, wherein the ratio of PPI or PEI to the agent(s) is about 0.5 : 1 to about 10 : 1, preferably wherein said ratio is about 1 : 1 to about 5 : 1.
5. A polypropylenimine-dendrimer (PPI) or a polyethylenimine (PEI), wherein said PPI or said PEI comprises one or more N-Boc-tyrosine residue(s), and wherein said PEI is a linear PEI or a branched PEI.
6. The composition according to any one of items 1 to 4, or the PPI or the PEI according to item 5, wherein between about 20% and about 80%, preferably between about 40% and about 70% of all terminal amino groups of said PPI are linked to an N-Boc-tyrosine residue, wherein between about 1% and about 64%, preferably between about 3% and about 15% of all terminal amino groups of said branched PEI are linked to an N-Boc-tyrosine residue and/or wherein between about 1% and about 60%, preferably between about 10% and about 20% of all nitrogen groups of said linear PEI are linked to an N-Boc-tyrosine residue.
7. The composition according to any one of items 1 to 4 and 6, or the PPI or the PEI according to item 5 or 6, wherein said PPI or said PEI further comprises one or more tyrosine residue(s), preferably wherein said one or more tyrosine residue(s) are each linked to one or more terminal amino group(s) of said PPI or to one or more amino group(s) of said branched PEI or to one or more nitrogen group(s) of said linear PEI.
8. The composition according to any one of items 1 to 4, 6 and 7, or the PPI or the PEI according to any one of item 5 to 7, wherein between about 0% and about 80%, preferably between about 30% and about 60% of all terminal amino groups of said PPI are linked to a tyrosine residue, wherein between about 2% and about 57% of all terminal amino groups of said branched PEI are linked to a tyrosine residue, and wherein between about 0% and about 57% of all nitrogen groups of said linear PEI are linked to a tyrosine residue.
9. The composition according to any one of items 1 to 4, and 6 to 8, or the PPI according to any one of items 5 to 8, wherein said PPI is a PPI of the second, third, or fourth generation, preferably of the third or the fourth generation.
10. The composition according to item 9, or the PPI according to item 9, wherein said PPI is a PPI of the third generation.
11. The composition according to item 9, or the PPI according to item 9, wherein said PPI is a PPI of the fourth generation.
12. The composition according to any one of items 9 to 11, or the PPI according to any one of items 9 to 11, wherein about 60% of all terminal amino groups of said PPI are linked to an N-Boc-tyrosine residue, and wherein about 0% of all terminal amino groups of said PPI are linked to a tyrosine residue.
13. The composition according to any one of items 9 to 11, or the PPI according to any one of items 9 to 11, wherein about 60% of all terminal amino groups of said PPI are linked to an N-Boc-tyrosine residue, and wherein about 40% of all terminal amino groups of said PPI are linked to a tyrosine residue.
14. The composition according to any one of items 9 to 11, or the PPI according to any one of items 9 to 11, wherein about 30% of all terminal amino groups of said PPI are linked to an N-Boc-tyrosine residue, and optionally wherein about 70% of all terminal amino groups of said PPI are linked to a tyrosine residue.
15. The composition according to any one of items 1 to 4, and 6 to 8, or the PEI according to any one of items 5 to 8, wherein said PEI has a molecular weight between about 2 kDa and about 25 kDa, preferably between about 5 kDa and about 15 kDa.
16. The composition according to any one of items 1 to 4, 6 to 8 and 15, or the PEI according to any one of items 5 to 8 and 15, wherein about 3% to about 15% of all terminal amino groups of said branched PEI are linked to an N-Boc-tyrosine residue, and wherein about 15% to about 27% of all terminal amino groups of said branched PEI are linked to a tyrosine residue.
17. The composition according to any one of items 1 to 4, 6 to 8, 15, and 16, or the PEI according to any one of items 5 to 8, 15, and 16, wherein about 18% of all nitrogen groups of said linear PEI are linked to an N-Boc-tyrosine residue, and wherein about 18% of all amino groups of said linear PEI are linked to a tyrosine residue.
18. The composition according to any one of items 1 to 4 and 6 to 17, wherein said one or more agent(s) are one or more therapeutic agent(s), one or more diagnostic agent(s), and/or one or more cosmetic agent(s).
19. The composition according to item 18, wherein the composition is a pharmaceutical composition, optionally comprising a pharmaceutically acceptable carrier, diluent and/or excipient.
20. The composition according to item 18 or 19 for use as a medicament.
21. The composition according to item 18 or 19 for use in the treatment, the prevention, and/or the diagnosis of a disease.
22. A method of administering the composition according to any one of items 1 to 4 and 6 to 19 to a subject.
23. Use of the composition according to any one of items 1 to 4 and 6 to 19, in the transfection of a cell *in vitro* or *ex vivo.*
24. A method for the transfection of a cell *in vitro* or *ex vivo,* the method comprising contacting said cell with the composition according to any one of items 1 to 4 and 6 to 19.
25. A method for the manufacture of a PPI according to any one of items 5 to 14 or a PEI according to any one of items 5 to 8 and 15 to 17.
26. A method for the manufacture of a composition according to any one of items 1 to 4 and 6 to 19, wherein the composition comprises one or more agents(s) as defined in any of the preceding items.

In a first aspect, the present invention provides for a polypropylenimine-dendrimer (PPI), wherein said PPI comprises one or more N-Boc-tyrosine residue(s). As indicated above, and illustratively shown in the enclosed examples and figures, the present inventors surprisingly found that such PPIs are particularly advantageous, *inter alia,* in the transfection of a cell *in vitro* or *in vivo.*

In the context of the present invention, said one or more N-Boc tyrosine residue(s) (also termed Boc residue(s) herein) are covalently linked/coupled/fused to said PPI. In particular, said one or more Boc residue(s) are covalently linked to terminal amino groups of said PPI. See, for example **Formulas I** and **II** illustrating exemplary PPIs comprising one or more Boc residue(s).

In the context of the present invention, preferably less than about 100% of all terminal amino groups of said PPI are linked to an N-Boc-tyrosine residue. In other words, it is herein preferred that the herein provided PPIs do not comprise a Boc residue on every terminal amino group. More preferably less than about 99%, even more preferably less than about 95%, even more preferably less than about 90%, even more preferably less than about 85%, even more preferably less than about 84%, even more preferably less than about 83%, even more preferably less than about 82%, even more preferably less than about 81%, even more preferably less than about 80% of all terminal amino groups of said PPI are linked to an N-Boc-tyrosine.

It is further preferred herein that at least about 5%, more preferred at least about 10%, even more preferred at least about 15%, even more preferred at least about 16%, even more preferred at least about 17%, even more preferred at least about 18%, even more preferred at least about 19%, even more preferred at least about 20% of all terminal amino groups of said PPI are linked to an N-Boc-tyrosine residue.

The herein provided PPI may comprise between about 20% and about 80% of all terminal amino groups of said PPI linked to an N-Boc-tyrosine residue. The herein provided PPI may preferably comprise between about 25% and about 75%, more preferably between about 30% and about 75%, even more preferably between about 35% and about 75%, even more preferably between about 36% and about 74%, even more preferably between about 37% and about 73%, even more preferably between about 38% and about 72%, even more preferably between about 39% and about 71%, even more preferably between about 40% and about 70%, of all terminal amino groups of said PPI are linked to an N-Boc-tyrosine residue. The herein provided PPI may preferably comprise between about 41% and about 69%, more preferably between about 45% and about 69%, even more preferably between about 46% and about 69%, even more preferably between about 47% and about 69%, even more preferably between about 48% and about 69%, even more preferably between about 49% and about 69%, even more preferably between about 50% and about 69%, even more preferably between about 51% and about 69%, even more preferably between about 52% and about 68%, even more preferably between about 53% and about 67%, even more preferably between about 54% and about 66%, even more preferably between about 55% and about 65%, even more preferably between about 56% and about 64%, even more preferably between about 57% and about 63%, even more preferably between about 58% and about 62%, even more preferably between about 59% and about 61%, even more preferably about 60% of all terminal amino groups of said PPI are linked to an N-Boc-tyrosine residue. In a preferred embodiment, the present invention provides for a PPI comprising about 60% of all terminal amino groups of said PPI are linked to an N-Boc-tyrosine residue.

The present inventors have further provided for double modified PPIs carrying/comprising one or more Boc residue(s) and one or more tyrosine residue(s), as well as for inventive means and methods for their production/manufacture. Such double modified PPIs were herein also shown to be of particular advantage in *in vitro* or *in vivo* transfection of a cell. Accordingly, the present invention also provides for a PPI comprising one or more N-Boc-tyrosine residue(s), wherein said PPI further comprises one or more tyrosine residue(s). The herein provided double modified PPIs *(i.e.,* carrying/comprising one or more Boc residue(s) and one or more tyrosine residue(s)) are preferred in the context of the present invention.

In combination/in sum/in total, said Boc residues and said tyrosine residues may be linked to up to and including 100% of all terminal amino groups of said PPI (e.g., with 100% corresponding to a single Boc or tyrosine modification on every terminal amino group of said PPI). The terminal amino groups or nitrogen groups (of the herein provided PPI) that are not coupled to Boc, tyrosine, or Fmoc may or may not be modified by other means, preferably they are not modified. It is further envisaged herein that a terminal amino group of said PPI can be coupled/linked to one or more Boc residue(s) and/or one or more tyrosine residue(s) and/or one or more other modification(s), preferably to one or more Boc residue(s) and/or one or more tyrosine residue(s), more preferably each terminal amino group of said PPI comprises only a single modification (e.g., a single Boc residue or a single tyrosine residue).

Preferably, between about 20% to about 100%, more preferably between about 30% to about 100% of all terminal amino groups of said PPI are coupled to a Boc residue or to a tyrosine residue, wherein said PPI comprises at least one Boc residue.

It is preferred that the herein provided PPIs comprise more Boc residues than tyrosine residues (linked to said terminal amino groups of said PPI).

In the context of the present invention, between about 0% and about 80%, preferably between about 30% and about 60% of all terminal amino groups of said PPI may be linked to a tyrosine residue. Accordingly, herein provided is a PPI, wherein between about 20% and about 80% of all terminal amino groups of said PPI are linked to an N-Boc-tyrosine residue and wherein between about 0% and about 80%, of all terminal amino groups of said PPI are linked to a tyrosine residue. Further herein provided is a PPI, wherein between about 40% and about 70% of all terminal amino groups of said PPI are linked to an N-Boc-tyrosine residue and wherein about 30% and about 60% of all terminal amino groups of said PPI are linked to a tyrosine residue.

The skilled person is aware of unmodified PPIs of different complexities and is, thus, aware of different generations of PPIs. For example, **Formulas I** and **II** illustratively show a PPI of the third and the fourth generation, respectively. The herein provided PPI may be a PPI of the second, third, or fourth generation, preferably of the third or the fourth generation.

The herein provided PPI may preferably be a PPI of the third generation. Accordingly, the herein provided PPI may be a PPI according to **Formula I.**

The herein provided PPI may preferably be a PPI of the fourth generation. Accordingly, the herein provided PPI may be a PPI according to **Formula II.**

In a particularly preferred embodiment, the present invention provides for a PPI, wherein about 60% of all terminal amino groups of said PPI are linked to an N-Boc-tyrosine residue, and wherein about 40% of all terminal amino groups of said PPI are linked to a tyrosine residue, preferably wherein said PPI is of the fourth generation.

In another embodiment, the present invention provides for a PPI, wherein about 60% of all terminal amino groups of said PPI are linked to an N-Boc-tyrosine residue, preferably wherein said PPI is of the third or the fourth generation, more preferably of the fourth generation.

In another particularly preferred embodiment, the present invention provides for a PPI, wherein about 60% of all terminal amino groups of said PPI are linked to an N-Boc-tyrosine residue, and wherein about 40% of all terminal amino groups of said PPI are linked to a tyrosine residue, preferably wherein said PPI is of the third generation.

In a further embodiment, the present invention provides for a PPI, wherein about 60% of all terminal amino groups of said PPI are linked to an N-Boc-tyrosine residue, and wherein about 0% of all terminal amino groups of said PPI are linked to a tyrosine residue, preferably wherein said PPI is of the third or the fourth generation, more preferably of the fourth generation.

In another embodiment, the present invention provides for a PPI, wherein about 30% of all terminal amino groups of said PPI are linked to an N-Boc-tyrosine residue, preferably wherein said PPI is of the third or the fourth generation, more preferably of the fourth generation.

In another embodiment, the present invention provides for a PPI, wherein about 30% of all terminal amino groups of said PPI are linked to an N-Boc-tyrosine residue, and wherein about 70% of all terminal amino groups of said PPI are linked to a tyrosine residue, preferably wherein said PPI is of the third or the fourth generation, more preferably of the fourth generation.

In another embodiment, the present invention provides for a PPI, wherein about 30% of all terminal amino groups of said PPI are linked to an N-Boc-tyrosine residue, and wherein about 0% of all terminal amino groups of said PPI are linked to a tyrosine residue, preferably wherein said PPI is of the third or the fourth generation, more preferably of the fourth generation.

In another aspect, the present invention provides for a polyethylenimine (PEI), wherein said PEI comprises one or more N-Boc-tyrosine residue(s). As indicated above, and illustratively shown in the enclosed examples and figures, the present inventors surprisingly found that such PEIs are particularly advantageous, *inter alia,* in the transfection of a cell *in vitro* or *in vivo.* The herein provided PEI may be a linear PEI or a branched PEI.

With regard to the herein provided branched PEIs, the one or more N-Boc-tyrosine residue(s) are covalently linked/coupled/fused/bound to said PEI. In particular, said one or more Boc residue(s) are covalently linked to terminal amino groups of said branched PEI. However, with regard to branched PEIs, Boc-residues may also be linked to other nitrogen groups of said branched PEI. See, for example **Formula III** illustrating an exemplary branched PEI comprising one or more Boc residue(s).

With regard to the herein provided linear PEIs, the one or more N-Boc-tyrosine residue(s) are covalently linked/coupled/fused/bound to said PEI. In particular, said one or more Boc residue(s) are covalently linked to nitrogen groups of said linear PEI. See, for example **Formula IV** illustrating an exemplary linear PEI comprising one or more Boc residue(s).

In the context of the present invention, preferably less than about 100%, more preferably less than about 90%, even more preferably less than about 80%, even more preferably less than about 70%, even more preferably less than about 65%, even more preferably about 64%, of all terminal amino groups of said branched PEI are linked to an N-Boc-tyrosine.

In the context of the present invention, preferably less than about 100%, more preferably less than about 90%, even more preferably less than about 80%, even more preferably less than about 70%, even more preferably less than about 65%, even more preferably about 60% of all nitrogen groups of said linear PEI are linked to an N-Boc-tyrosine.

It is further preferred herein that at least about 1%, more preferred at least about 1%, even more preferred at least about 1.5%, even more preferred at least about 2%, even more preferred at least about 2.5%, even more preferred about 3% of all terminal amino groups of said branched PEI are linked to an N-Boc-tyrosine residue.

It is further preferred herein that at least about 1%, even more preferred at least about 2%, even more preferred at least about 3%, even more preferred at least about 4%, even more preferred at least about 5%, even more preferred at least about 6%, even more preferred at least about 7%, even more preferred at least about 8%, even more preferred at least about 9%, even more preferred about 10% of all nitrogen groups of said linear PEI are linked to an N-Boc-tyrosine residue.

The herein provided branched PEI may comprise between about 1% and about 64%, preferably between about 1% and about 48% of all terminal amino groups of said branched PEI linked to an N-Boc-tyrosine residue. The herein provided branched PEI may preferably comprise between about 1% and about 45%, more preferably between about 1.5% and about 40%, more preferably between about 1.5% and about 35%, more preferably between about 1.5% and about 30%, more preferably between about 2% and about 25%, more preferably between about 2.5% and about 20%, more preferably between about 2.6% and about 19%, more preferably between about 2.7% and about 18%, more preferably between about 2.8% and about 17%, more preferably between about 2.9% and about 16%, more preferably between about 3% and about 15% of all terminal amino groups of said branched PEI linked to an N-Boc-tyrosine residue. In a preferred embodiment, the present invention provides for a branched PEI comprising about 3% of all terminal amino groups of said branched PEI are linked to an N-Boc-tyrosine residue.

The herein provided branched PEI may preferably comprise between about 1% and about 64%, more preferably between about 2% and about 48%, more preferably between about 3% and about 45%, more preferably between about 4% and about 40%, more preferably between about 5% and about 35%, more preferably between about 6% and about 30%, more preferably between about 7% and about 25%, more preferably between about 7.5% and about 20%, more preferably between about 8% and about 15% of all terminal amino groups of said branched PEI linked to an N-Boc-tyrosine residue.

In another preferred embodiment, the present invention provides for a branched PEI comprising about 8% of all terminal amino groups of said branched PEI are linked to an N-Boc-tyrosine residue. In another preferred embodiment, the present invention provides for a branched PEI comprising about 15% of all terminal amino groups of said branched PEI are linked to an N-Boc-tyrosine residue.

The herein provided linear PEI may comprise between about 1% and about 60%, preferably between about 10% and about 20% of all nitrogen groups of said linear PEI linked to an N-Boc-tyrosine residue. The herein provided linear PEI may preferably comprise between about 1% and about 60%, more preferably between about 2% and about 45%, more preferably between about 3% and about 40%, more preferably between about 4% and about 35%, more preferably between about 5% and about 30%, more preferably between about 6% and about 28%, more preferably between about 7% and about 26%, more preferably between about 8% and about 24%, more preferably between about 9% and about 22%, more preferably between about 10% and about 20% of all nitrogen groups of said linear PEI linked to an N-Boc-tyrosine residue.

The present inventors have further provided for double modified PEIs carrying/comprising one or more Boc residue(s) and one or more tyrosine residue(s), as well as for inventive means and methods for their production/manufacture. Such double modified PEIs were herein also shown to be of particular advantage in *in vitro* or *in vivo* transfection of a cell. Accordingly, the present invention also provides for a PEI comprising one or more N-Boc-tyrosine residue(s), wherein said PEI further comprises one or more tyrosine residue(s), preferably wherein said one or more tyrosine residue(s) are each linked to one or more amino group(s) of said branched PEI or to one or more nitrogen group(s) of said linear PEI. The herein provided double modified PEIs (*i.e*., carrying/comprising one or more Boc residue(s) and one or more tyrosine residue(s)) are preferred in the context of the present invention.

In combination/in sum/in total, said Boc residues and said tyrosine residues may be linked to up to and including 100% of all terminal amino groups of said branched PEI or of all nitrogen groups of said linear PEI (e.g., with 100% corresponding to a single Boc or tyrosine modification on every terminal amino group of said branched PEI or on every nitrogen group of said linear PEI.. The terminal amino groups or nitrogen groups (of the herein provided PEI) that are not coupled to Boc, tyrosine, or Fmoc may or may not be modified by other means, preferably they are not modified. It is further envisaged herein that a terminal amino group of said branched PEI or a nitrogen group of said linear PEI can be coupled/linked to one or more Boc residue(s) and/or one or more tyrosine residue(s) and/or one or more other modification(s), preferably to one or more Boc residue(s) and/or one or more tyrosine residue(s), more preferably each terminal amino group of said branched PEI or each nitrogen group of said linear PEI comprises only a single modification (e.g., a single Boc residue or a single tyrosine residue).

Preferably, between about 10% to about 60%, more preferably between about 15% to about 50%, more preferably between about 20% to about 40%, more preferably between about 25% to about 35%, more preferably about 30% of all terminal amino groups of said branched PEI are coupled to a Boc residue or to a tyrosine residue, wherein said branched PEI comprises at least one Boc residue.

Preferably, between about 10% to about 60%, more preferably between about 15% to about 40%, more preferably between about 20% to about 35%, more preferably about 35% of all nitrogen groups of said linear PEI are coupled to a Boc residue or to a tyrosine residue, wherein said linear PEI comprises at least one Boc residue.

It is preferred that the herein provided PEIs comprise more tyrosine residues than Boc residues (linked to said terminal amino groups of said branched PEI or linked to said nitrogen group of said linear PEI).

In the context of the present invention, between about 2% and about 57% of all terminal amino groups of said branched PEI may be linked to a tyrosine residue. Accordingly, herein provided is a branched PEI, wherein between about 1% and about 64% of all terminal amino groups of said branched PEI are linked to an N-Boc-tyrosine residue and wherein between about 2% and about 57% of all terminal amino groups of said branched PEI are linked to a tyrosine residue. Further herein provided is a branched PEI, wherein between about 1% and about 57% of all terminal amino groups of said branched PEI are linked to an N-Boc-tyrosine residue and wherein between about 1% and about 64% of all terminal amino groups of said branched PEI are linked to a tyrosine residue. Further herein provided is a branched PEI, wherein between about 1% and about 50% of all terminal amino groups of said branched PEI are linked to an N-Boc-tyrosine residue and wherein between about 3% and about 48% of all terminal amino groups of said branched PEI are linked to a tyrosine residue. Further herein provided is a branched PEI, wherein between about 1% and about 48% of all terminal amino groups of said branched PEI are linked to an N-Boc-tyrosine residue and wherein between about 2% and about 57% of all terminal amino groups of said branched PEI are linked to a tyrosine residue. Further herein provided is a branched PEI, wherein between about 2% and about 48% of all terminal amino groups of said branched PEI are linked to an N-Boc-tyrosine residue and wherein between about 5% and about 40% of all terminal amino groups of said branched PEI are linked to a tyrosine residue. Further herein provided is a branched PEI, wherein between about 3% and about 40% of all terminal amino groups of said branched PEI are linked to an N-Boc-tyrosine residue and wherein between about 7% and about 35% of all terminal amino groups of said branched PEI are linked to a tyrosine residue. Further herein provided is a branched PEI, wherein between about 5% and about 30% of all terminal amino groups of said branched PEI are linked to an N-Boc-tyrosine residue and wherein between about 9% and about 30% of all terminal amino groups of said branched PEI are linked to a tyrosine residue. Further herein provided is a branched PEI, wherein between about 6% and about 25% of all terminal amino groups of said branched PEI are linked to an N-Boc-tyrosine residue and wherein between about 11% and about 25% of all terminal amino groups of said branched PEI are linked to a tyrosine residue. Further herein provided is a branched PEI, wherein between about 7% and about 20% of all terminal amino groups of said branched PEI are linked to an N-Boc-tyrosine residue and wherein between about 13% and about 24% of all terminal amino groups of said branched PEI are linked to a tyrosine residue. Further herein provided is a branched PEI, wherein between about 8% and about 15% of all terminal amino groups of said branched PEI are linked to an N-Boc-tyrosine residue and wherein between about 15% and about 23% of all terminal amino groups of said branched PEI are linked to a tyrosine residue. Further herein provided is a preferred branched PEI, wherein between about 3% and about 15% of all terminal amino groups of said branched PEI are linked to an N-Boc-tyrosine residue and wherein between about 15% and about 27% of all terminal amino groups of said branched PEI are linked to a tyrosine residue, preferably wherein in sum said N-Boc-tyrosine residues and said tyrosine residues are linked to about 30% of all terminal amino groups of said branched PEI.

In the context of the present invention, between about 0% and about 57% of all nitrogen groups of said linear PEI may be linked to a tyrosine residue. Accordingly, herein provided is a linear PEI, wherein between about 1% and about 60% of all nitrogen groups of said linear PEI are linked to an N-Boc-tyrosine residue and wherein between about 0% and about 57% of all nitrogen groups of said linear PEI are linked to a tyrosine residue. Further herein provided is a linear PEI, wherein between about 2% and about 40% of all nitrogen groups of said linear PEI are linked to an N-Boc-tyrosine residue and wherein between about 0% and about 50% of all nitrogen groups of said linear PEI are linked to a tyrosine residue. Further herein provided is a linear PEI, wherein between about 3% and about 20% of all nitrogen groups of said linear PEI are linked to an N-Boc-tyrosine residue and wherein between about 0% and about 40% of all nitrogen groups of said linear PEI are linked to a tyrosine residue. Further herein provided is a linear PEI, wherein between about 5% and about 18% of all nitrogen groups of said linear PEI are linked to an N-Boc-tyrosine residue and wherein between about 0% and about 35% of all nitrogen groups of said linear PEI are linked to a tyrosine residue. Further herein provided is a linear PEI, wherein between about 6% and about 16% of all nitrogen groups of said linear PEI are linked to an N-Boc-tyrosine residue and wherein between about 5% and about 32% of all nitrogen groups of said linear PEI are linked to a tyrosine residue. Further herein provided is a linear PEI, wherein between about 7% and about 15% of all nitrogen groups of said linear PEI are linked to an N-Boc-tyrosine residue and wherein between about 10% and about 30% of all nitrogen groups of said linear PEI are linked to a tyrosine residue. Further herein provided is a linear PEI, wherein between about 8% and about 14% of all nitrogen groups of said linear PEI are linked to an N-Boc-tyrosine residue and wherein between about 15% and about 28% of all nitrogen groups of said linear PEI are linked to a tyrosine residue. Further herein provided is a linear PEI, wherein between about 9% and about 12% of all nitrogen groups of said linear PEI are linked to an N-Boc-tyrosine residue and wherein between about 18% and about 27% of all nitrogen groups of said linear PEI are linked to a tyrosine residue. Further herein provided is a linear PEI, wherein between about 10% and about 20% of all nitrogen groups of said linear PEI are linked to an N-Boc-tyrosine residue and wherein between about 0% and about 57% of all nitrogen groups of said linear PEI are linked to a tyrosine residue. Further herein provided is a preferred linear PEI, wherein between about 10% and about 20% of all nitrogen groups of said linear PEI are linked to an N-Boc-tyrosine residue and wherein between about 0% and about 18% of all nitrogen groups of said linear PEI are linked to a tyrosine residue. Further herein provided is a preferred linear PEI, wherein about 18% of all nitrogen groups of said linear PEI are linked to an N-Boc-tyrosine residue and wherein about 18% of all nitrogen groups of said linear PEI are linked to a tyrosine residue. Further herein provided is a preferred linear PEI, wherein about 20% of all nitrogen groups of said linear PEI are linked to an N-Boc-tyrosine residue and wherein about 0% of all nitrogen groups of said linear PEI are linked to a tyrosine residue. Further herein provided is a preferred linear PEI, wherein about 10% of all nitrogen groups of said linear PEI are linked to an N-Boc-tyrosine residue and wherein about 0%of all nitrogen groups of said linear PEI are linked to a tyrosine residue.

The herein provided PEIs may have a molecular weight between about 2 kDa and about 25 kDa, preferably between about 5 kDa and about 15 kDa, more preferably between about 5 kDa and about 10 kDa, even more preferably about 10 kDa. Such molecular weight values refer to a non-modified PEI (i.e., to a PEI without N-Boc-tyrosine residues and tyrosine residues). The skilled person can readily determine the molecular weight of a PEI comprising one or more N-Boc-tyrosine residue(s) and/or one or more tyrosine residue(s) based on the degree of modification of said PEI. The branching degree of the herein provided branched PEIs is not so limited, and can be readily adapted by the skilled person.

In a preferred embodiment, the present invention provides for a branched PEI, wherein about 15% of all terminal amino groups of said branched PEI are linked to an N-Boc-tyrosine residue, and wherein about 15% of all terminal amino groups of said branched PEI are linked to a tyrosine residue, preferably wherein said PEI without said N-Boc-tyrosine residues and said tyrosine residues has a molecular weight of preferably about 10 kDa.

In another preferred embodiment, the present invention provides for a branched PEI, wherein about 3% of all terminal amino groups of said branched PEI are linked to an N-Boc-tyrosine residue, and wherein about 27% of all terminal amino groups of said branched PEI are linked to a tyrosine residue, preferably wherein said PEI without said N-Boc-tyrosine residues and said tyrosine residues has a molecular weight of preferably about 10 kDa.

The present invention further provides for compositions and nanoparticles comprising the herein provided polypropylenimine-dendrimer (PPI) comprising one or more N-Boc-tyrosine residue(s) and the herein provided polyethylenimine (PEI) comprising one or more N-Boc-tyrosine residue(s). Accordingly, the compounds comprised in the herein provided compositions or nanoparticles may be any one or more of the herein above detailed polypropylenimine-dendrimers (PPI) and/or polyethylenimines (PEI).

Accordingly, in another aspect, the present invention provides for a composition comprising a polypropylenimine-dendrimer (PPI) or a polyethylenimine (PEI), wherein said PPI or said PEI comprises one or more N-Boc-tyrosine residue(s), and optionally wherein the composition further comprises one or more agent(s).

The composition of the present invention may comprise nanoparticles comprising said PPI or said PEI and optionally said agent. Accordingly, said PPI or said PEI may form a nanoparticle with said one or more agent(s) and/or may be formulated as a nanoparticle with said one or more agent(s). Accordingly, the present invention also provides for a nanoparticle comprising said PPI or said PEI and said agent.

Said agent(s) may be one or more polynucleotide molecule(s), one or more polypeptide(s), and/or one or more small molecule(s), preferably one or more polynucleotide molecule(s).

Said agent(s) may be one or more polynucleotide molecule(s) selected from the group consisting of DNA, RNA, mRNA, self-amplifying mRNA (sa-mRNA), minicircle DNA, DNA plasmid, small inhibitory RNA (siRNA), single guide RNA (sgRNA), guide RNA (gRNA), microRNA (miRNA), anti-miRNA (antimir), Gapmer, and circular RNA (circRNA). Said one or more agent(s) may be a single polynucleotide molecule selected from the above.

An antimiR is a single-stranded polynucleotide, which may be composed of DNA, RNA, or analogues or derivatives thereof, preferably RNA, that is designed to be at least partially complementary to a target microRNA (miRNA) sequence and is capable of inhibiting the expression and/or function of the target miRNA, including by binding to at least the seed region of the mature miRNA sequence, thereby preventing the miRNA from interacting with its natural mRNA targets.

A Gapmer is a polynucleotide comprising a central region of consecutive DNA nucleotides (the "gap") flanked on both sides by regions of chemically modified nucleotides or RNA nucleotides, wherein the central DNA region is capable of recruiting RNase H upon hybridization to a target RNA, resulting in cleavage of the RNA strand, and the flanking regions confer increased nuclease resistance and binding affinity to the target sequence.

A circRNA (circular RNA) is a covalently closed, single-stranded RNA molecule that lacks free 5' and 3' ends, it may function as a non-coding RNA (e.g., providing specific binding sites for a miRNA) or encode a polypeptide, and usually has increased stability relative to linear RNA due to its circular structure.

A gRNA (guide RNA) is a synthetic or naturally occurring RNA molecule that comprises a sequence region complementary to a target nucleic acid and a structural region capable of associating with a nuclease or editing complex, thereby directing the complex (e.g., a CAS endonuclease) to a specific genomic or transcriptomic locus for e.g., site-specific genome editing.

A sgRNA (single guide RNA) is a chimeric RNA molecule that combines the functions of a gRNA and a trans-activating RNA into a single transcript, containing a target-specific region and a scaffold region for binding to a CRISPR-associated (Cas) protein, thereby enabling e.g., site-specific genome editing.

Minicircle DNA is a double-stranded, circular DNA molecule that contains an expression cassette or gene of interest but is substantially free of bacterial plasmid backbone sequences (including origins of replication and antibiotic resistance genes), thus is smaller as compared to a plasmid, thereby may provide improved transgene expression, reduced immunogenicity, and/or increased safety for gene delivery applications (as compared to e.g., plasmids).

sa-mRNA (self-amplifying mRNA) is a messenger RNA molecule that encodes means such as an RNA-dependent polymerase (e.g., a viral replicase), allowing for the replication of said sa-RNA in the transfected cell.

Said DNA, RNA, mRNA, said sa-mRNA, said minicircle DNA, and said DNA plasmid may encode one or more polypeptide(s).

Said polynucleotides may or may not comprise chemical modifications. The enclosed examples illustratively show that the herein provided compositions can *e.g*., effectively induce expression of a polypeptide encoded by a polynucleotide molecule comprised therein even when no chemical modification were introduced to said polynucleotide molecule. The skilled artisan is aware of chemical modifications and means and methods for introducing the same that may further enhance, *e.g.,* expression of an encoded polypeptide. Such chemical modification may include, without limitation, phosphorothioate, 2'-O-Methylation, LNA-(Locked Nucleic Acid), 2'-O-Methoxy-, 2'-Fluoro-, 2'-O-(2-Methoxyethyl)--, 2'-Fluoroarabino-, and UNA- (Unlocked Nucleic Acid) modifications and/or base analog substitutions such as, for example, N1-Methyl-Pseudouridine, 5, 5-Bromouracil, 2-Aminopurine, Isoguanine, 5-Methylisocytosine, Inosine, 5-Nitroindole, 3-Nitropyrrole, 7-Deaza-Guanine, 7-Deaza-Adenine, Tricyclic Cytosine, Cordycepin, 4-Nitrobenzimidazole.

In a preferred embodiment, the present invention provides for a composition comprising said PPI or said PEI and one or more agent(s), wherein said agent(s) is/are selected from the group consisting of DNA, RNA, mRNA, self-amplifying mRNA (sa-mRNA), minicircle DNA, DNA plasmid, small inhibitory RNA (siRNA), and single guide RNA (sgRNA).

In another preferred embodiment, the present invention provides for a composition comprising said PPI or said PEI and one or more agent(s), wherein said agent(s) is/are selected from the group consisting of mRNA, self-amplifying mRNA (sa-mRNA), minicircle DNA, single guide RNA (sgRNA), microRNA (miRNA), anti-miRNA (antimir), Gapmer, and circular RNA (circRNA).

In another preferred embodiment, the present invention provides for a composition comprising said PPI or said PEI and one or more agent(s), wherein said agent(s) is/are selected from the group consisting of DNA, RNA, mRNA, self-amplifying mRNA (sa-mRNA), minicircle DNA, DNA plasmid, and single guide RNA (sgRNA).

As indicated above, the herein provided PPIs/PEIs (and compositions/nanoparticles comprising the same) are, as opposed to the PPIs/PEIs known from the state of the art, particularly useful in the transfection of long nucleic acids. Accordingly, in another preferred embodiment, the present invention provides for a composition comprising said PPI or said PEI and one or more agent(s), wherein said agent(s) is/are selected from the group consisting of mRNA, self-amplifying mRNA (sa-mRNA), and minicircle DNA, DNA plasmid.

The herein provided PPIs/PEIs (and compositions/nanoparticles comprising the same) are surprisingly particularly useful in the transfection of mRNA (as illustrated, e.g., in Example 10). Accordingly, in a further preferred embodiment, the present invention provides for a composition comprising said PPI or said PEI and one or more agent(s), with said agent(s) being mRNA.

The ratio of said PPI or said PEI to said agent(s) in said composition may be about 0.5 : 1 to about 10 : 1, preferably wherein said ratio is about 1 : 1 to about 5 : 1. The ratio of said PPI or said PEI to said agent(s) in said composition may be about 2.5 : 1, about 3.75 : 1, and about 5 : 1, preferably about 3.75 : 1 or about 5 : 1. The skilled person can readily further optimize the ratio of said PPI or said PEI to said agent.

The composition may further comprise a buffer, buffer solution, and/or a buffer system. The nanoparticle may also further comprise a buffer, buffer solution, and/or a buffer system and/or may be comprised in a buffer, buffer solution, and/or a buffer system. Such buffers, buffer solutions, and/or buffer systems may comprise an inorganic salt (*e.g.* NaCl), an organic acid (*e.g*., carboxylic acids or sulfonic acids such as citric acid or HEPES), and/or a carbohydrate (*e.g*. glucose, trehalose). Such buffers, buffer solutions, and/or buffer systems preferably have a physiological pH, preferably a pH between 4.5 and 8.

The above-described agent(s) may be one or more therapeutic agent(s), one or more diagnostic agent(s), and/or one or more cosmetic agent(s), preferably one or more therapeutic agent(s). Said therapeutic agent(s) may be therapeutic nucleic acid(s), preferably one or more nucleic acid(s) each encoding one or more therapeutic polypeptide(s). Accordingly, said composition may be a pharmaceutical composition, optionally comprising a pharmaceutically acceptable carrier, diluent and/or excipient.

The present invention further provides for the above-described (pharmaceutical) composition for use as a medicament in a subject. The present invention further provides for the above-described (pharmaceutical) composition for use in the treatment, the prevention, and/or the diagnosis of a disease in a subject.

The present invention further provides for a method of administering the herein provided composition to a subject.

The present invention further provides for a use of the herein provided composition in the transfection of a cell *in vitro* or *ex vivo.* Such cells may be prokaryotic or eukaryotic cells.

In a preferred embodiment, the present invention provides for a composition comprising said PPI or said PEI and one or more agent(s), wherein said agent(s) are an sgRNA and an mRNA encoding a genome editing endonuclease. Such a genome editing endonuclease may be a Cas endonuclease, a zinc finger nuclease, a TALEN, or the like, preferably a Cas endonuclease. In the context of the present invention, the Cas endonuclease is not particularly limited and can be any Cas protein mediating gene editing, base editing, prime editing or a similar process, preferably the Cas endonuclease is Cas9. The present invention further provides for a use of said composition for inducing a mutation in a transfected cell *in vitro* or *ex vivo.* The present invention further provides for said composition for use in the treatment of a disease by inducing a mutation in a transfected subject, wherein said agent(s) is/are one or more therapeutic agent(s).

In another preferred embodiment, the present invention provides for a composition comprising said PPI or said PEI and one or more agent(s), wherein said agent is mRNA or sa-mRNA. The present invention further provides for a use of said composition in inducing the expression of one or more polypeptide(s) encoded by said mRNA or said sa-mRNA in a transfected cell *in vitro* or *ex vivo.* The present invention further provides for said composition for use in the treatment of a disease by inducing the expression of one or more polypeptide(s) encoded by said mRNA or said sa-mRNA in a transfected subject, wherein said agent(s) is/are one or more therapeutic agent(s).

In another preferred embodiment, the present invention provides for a composition comprising said PPI or said PEI and one or more agent(s), wherein said agent is DNA, preferably minicircle DNA or a DNA plasmid. The present invention further provides for a use of said composition in inducing the expression one or more polypeptide(s) encoded by said DNA in a transfected cell *in vitro* or *ex vivo.* The present invention further provides for said composition for use in the treatment of a disease by inducing the expression of one or more polypeptide(s) encoded by said DNA in a transfected subject, wherein said agent(s) is/are one or more therapeutic agent(s).

In another preferred embodiment, the present invention provides for a composition comprising said PPI or said PEI and one or more agent(s), wherein said agent is an siRNA. The present invention further provides for a use of said composition in inducing the knockdown of a gene in a transfected cell *in vitro* or *ex vivo.* The present invention further provides for said composition for use in the treatment of a disease by inducing the knockdown of a gene in a transfected subject, wherein said agent(s) is/are one or more therapeutic agent(s).

In another aspect, the present invention provides for a kit comprising the herein provided PPI and/or the herein provided PEI. Said kit may further comprise a buffer, buffer solution, and/or a buffer. Such buffers, buffer solutions, and/or buffer systems may be defined as herein above. Said kit further comprise any of the herein specified agents. Said kit may be for use in complexing/formulating an agent with any of the herein provided PPI or PEI. Said kit may be for use in forming/formulating a nanoparticle comprising any of the herein provided PPI or PEI and an agent, as defined herein above. Said kit may be for use in in the transfection of a cell with an agent, as defined herein above.

The present invention further provides for means and methods for the manufacture/production of the herein provided PPI or of the herein provided PEI.

**Example 1.B** illustratively demonstrates the production of various PPIs and PEIs and indicates the employed concentrations of *e.g*., N-Boc-tyrosine, Fmoc-tyrosine, and the to be modified PPIs or PEIs. Based on the information provided in **Example 1.B**, the skilled person can readily determine concentrations and conditions to produce a Boc- and/or tyrosine-modified PPI or a Boc- and/or tyrosine-modified PEI having desired degrees of modifications.

In an embodiment of said method, the to be produced PPI is a PPI comprising one or more N-Boc-tyrosine residue(s) and one or more tyrosine residue(s) or the to be produced PEI is a PEI comprising one or more N-Boc-tyrosine residue(s) and one or more tyrosine residue(s), wherein the method comprises the steps of:
(i) reacting a PPI or a PEI with N-Boc-tyrosine and with a further N-terminally protected-tyrosine residue;
(ii) deprotecting said further N-terminally protected-tyrosine residue.

Accordingly, said method produces or is capable of producing a double-modified PPI or a double-modified PEI.

Said further N-terminally protected-tyrosine residue may be a tyrosine residue N-terminally protected by an acid-stable amine protection group. Said acid-stable amine protection group may be a N-terminal protection group selected from the group consisting of Fmoc, Smoc (2,7-disulfo-9-fluorenylmethoxycarbonyl), TFA (Trifluoromethylacetyl), Dde (1-(4,4-dimethyl-2,6-dioxocyclohex-1-ylidene)ethyl), Teoc (2-(trimethylsilyl)ethoxycarbonyl), pNB (para-Nitrobenzyl), and Nosyl (Nitrobenzensulfonyl), preferably Fmoc.

The PPI or the PEI to be reacted in step (i) preferably is a PPI or a PEI not comprising N-Boc-tyrosine or Fmoc-tyrosine (e.g., a PPI or PEI according to **Formulas I** to **IV,** wherein 'R' is hydrogen). Reacting a PPI or a PEI with N-Boc-tyrosine and with Fmoc-tyrosine in step (i) thereby couples said N-Boc-tyrosine and said Fmoc-tyrosine with said PPI or said PEI.

Deprotecting said Fmoc-tyrosine in step (ii) removes a Fmoc-protection group, thereby forming a tyrosine residue (coupled to said PPI or PEI).

Said method may further comprise step (iii), wherein step (iii) comprises obtaining said to be produced PPI or PEI. The present invention further provides for a PPI or a PEI obtained or obtainable by said method.

The herein provided PPI or PEI (in particular the herein provided mono-modified PPI or PEI) may further comprise one or more Fmoc-tyrosine residue(s).

Accordingly, the present invention further provides for a PPI comprising one or more N-Boc-tyrosine residue(s) and one or more Fmoc-tyrosine residue(s).

The present invention further provides for a method for the manufacture/production of the herein provided composition.

Said method may comprise steps (i) to (v):
(i) preparing or obtaining a first composition comprising one or more agent(s) (preferably one or more polynucleotide molecule(s)) in a buffer solution (preferably, said buffer solution is a buffer solution as defined herein above),
(ii) preparing or obtaining a second composition comprising the herein provided PPI or the herein provided PEI in a buffer solution (preferably, said buffer solution is a buffer solution as defined herein above),
(iii) mixing of said first and said second composition,
(iv) incubating the composition of step (iii), preferably for about 15 minutes to about 1.5 hours, more preferably for about 30 to about 45 minutes, and
(v) optionally, processing the composition of step (iv) for storage, optionally by freezing, lyophilization or spray-drying.

Any one of steps (i) to (iv) may be performed at room temperature.

Said method may further comprise a step of obtaining the composition of step (iv) or (v). The present invention further provides composition obtained or obtainable by said method.

### Brief description of the drawings

The present invention is further described by reference to the following non-limiting figures. The ratios (xx/zz) (e.g. (30/70), (60/40), (0/100), (30/0) as used herein refer to the ratio of N-Boc-tyrosine/tyrosine (percentage of (N-Boc-tyrosine of all terminal amino groups per PPI)/(tyrosine linked to terminal amino groups per PPI). The figures show:
**Fig. 1** **Efficacy of PPI-G4-Y(60/40) for mRNA transfection.** (A) Time-dependent EGFP expression, as determined by total integrated fluorescence intensity, after transfection of PC3 cells with PPI-G4-Y(60/40)/mRNA complexes/nanoparticles prepared at different polymer/mRNA mass ratios as indicated. Results are compared to mono-modified PPI-G4-Y(0/100) previously described in the literature as PPI-Y and to jetMessenger^{®} (JM). (B) Comparison of transfection efficacies, as determined by mean fluorescence intensities of EGFP positive cells, after using different mRNA amounts and complexed at different polymer/mRNA mass ratios as indicated. Bars represent standard deviations (SD).

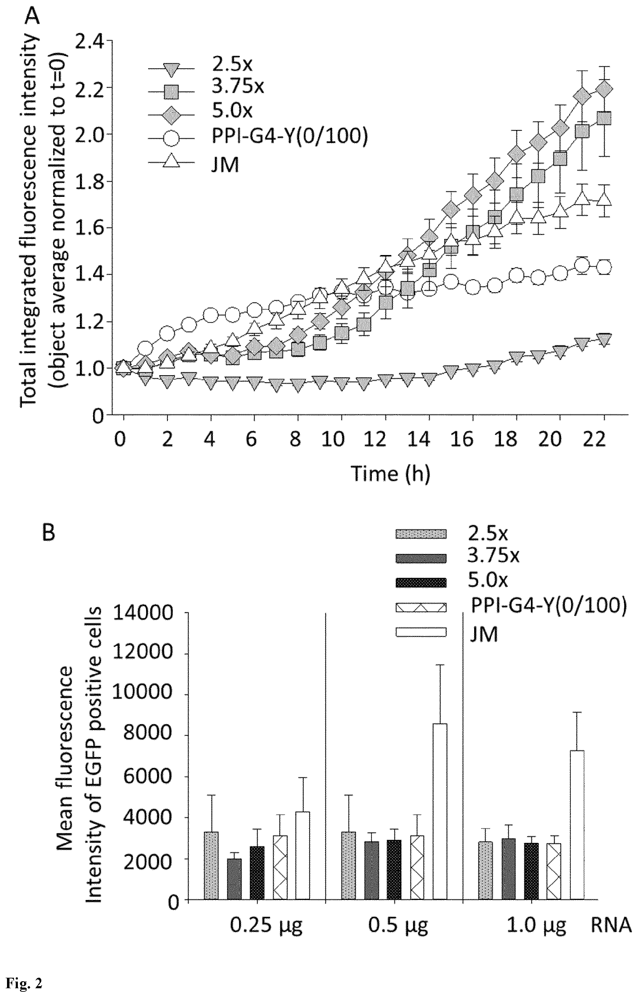

**Fig. 2** **Efficacy of PPI-G4-Y(30/70) for mRNA transfection.** (A) Time-dependent EGFP expression, as determined by total integrated fluorescence intensity, after transfection of PC3 cells with PPI-G4-Y(30/70)/mRNA complexes/nanoparticles prepared at different polymer/mRNA mass ratios as indicated. Results are compared to mono-modified PPI-G4-Y(0/100) previously described in the literature as PPI-Y and to jetMessenger^{®} (JM). (B) Comparison of transfection efficacies, as determined by mean fluorescence intensities of EGFP positive cells, after using different mRNA amounts and complexed at different polymer/mRNA mass ratios as indicated. Bars represent standard deviations (SD).

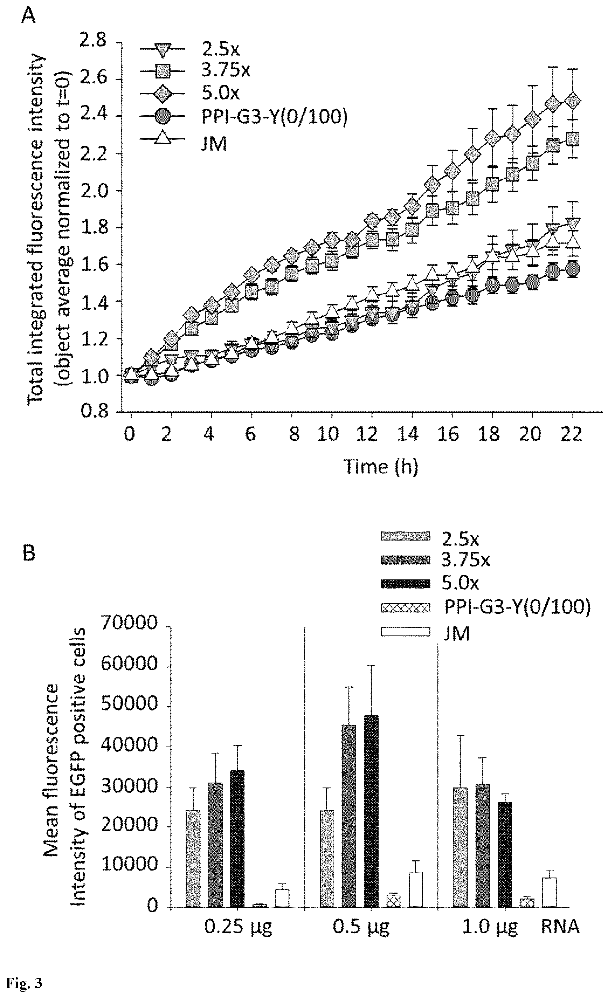

**Fig. 3** **Efficacy of PPI-G3-Y(60/0) for mRNA transfection.** (A) Time-dependent EGFP expression, as determined by total integrated fluorescence intensity, after transfection of PC3 cells with PPI-G3-Y(60/0)/mRNA complexes/nanoparticles prepared at different polymer/mRNA mass ratios as indicated. Results are compared to mono-modified PPI-G3-Y(0/100) previously described in the literature as PPI-Y and to jetMessenger^{®} (JM). (B) Comparison of transfection efficacies, as determined by mean fluorescence intensities of EGFP positive cells, after using different mRNA amounts and complexed at different polymer/mRNA mass ratios as indicated. Bars represent standard deviations (SD).

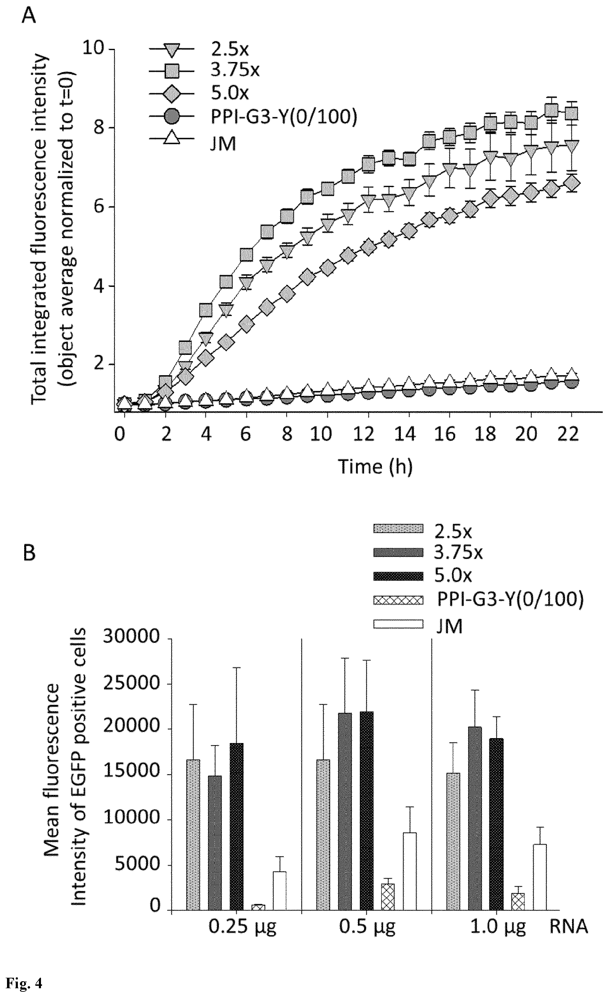

**Fig. 4** **Efficacy of PPI-G3-Y(60/40) for mRNA transfection.** (A) Time-dependent EGFP expression, as determined by total integrated fluorescence intensity, after transfection of PC3 cells with PPI-G3-Y(60/40)/mRNA complexes/nanoparticles prepared at different polymer/mRNA mass ratios as indicated. Results are compared to mono-modified PPI-G3-Y(0/100) previously described in the literature as PPI-Y and to jetMessenger^{®} (JM). (B) Comparison of transfection efficacies, as determined by mean fluorescence intensities of EGFP positive cells, after using different mRNA amounts and complexed at different polymer/mRNA mass ratios as indicated. Bars represent standard deviations (SD).
**Fig. 5** **Efficacy of different PPIY derivatives for the transfection of self-amplifying mRNA (sa-mRNA).** (A) Time-dependent EGFP expression, as determined by total green object area, after transfection of PC3 cells with complexes/nanoparticles based on the dendrimers indicated in the figure and containing sa-mRNA. Results are compared to jetMessenger^{®} (JM). Bars represent standard deviations (SD). (B) Microscopic picture of two cell colonies after 48 days of cultivation with multiple splitting rounds. In bright field, two colonies are seen (right). While only one colony is EGFP positive as visible in fluorescence microscopy, all daughter cells show high EGFP expression (left).

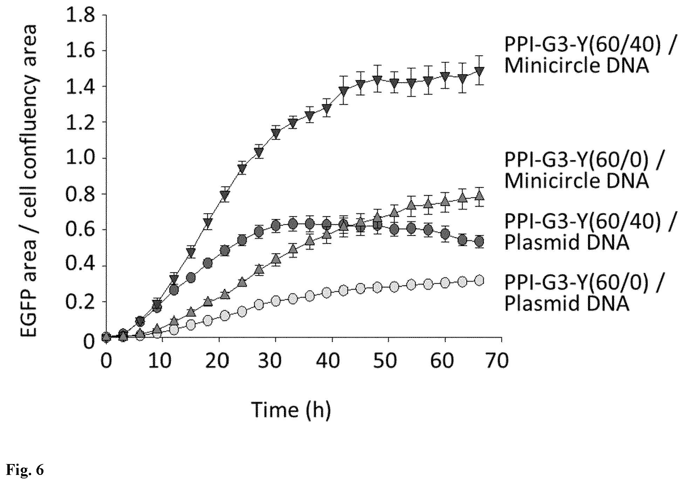

**Fig. 6** **Efficacy of different PPI-G3-Y derivatives for the transfection of minicircle DNA vs. plasmid DNA.** Time-dependent EGFP expression, as determined by EGFP area / cell confluency area, after transfection of PC3 cells with complexes/nanoparticles based on PPI-G3-Y(60/40) or PPI-G3-Y(60/0) and containing minicircle or plasmid DNA as indicated in the figure. Bars represent standard deviations (SD).

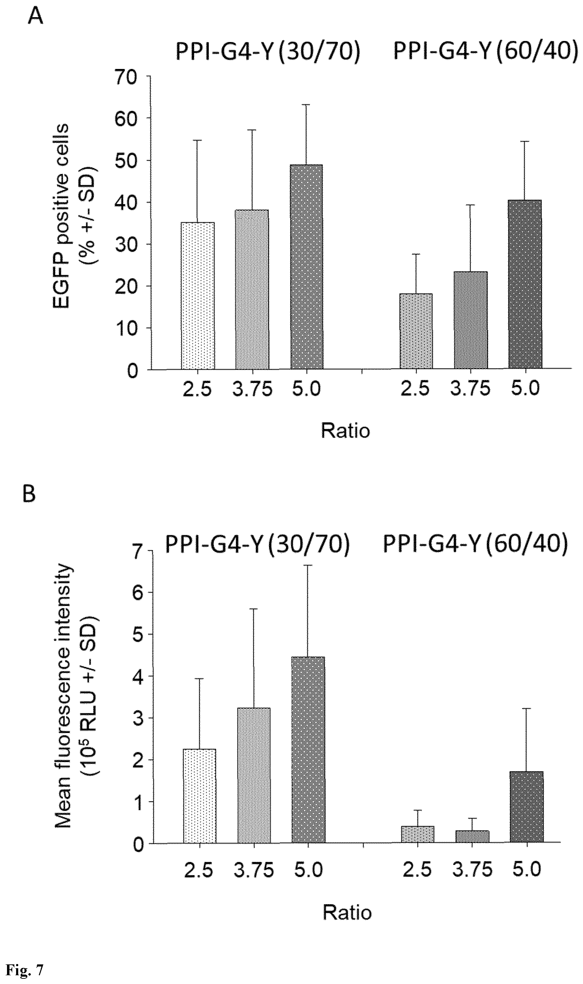

**Fig. 7** **Efficacy of different PPI-G4-Y derivatives for the transfection of plasmid DNA.** (A) Percentage of EGFP-positive PC3 cells after their transfection with complexes/nanoparticles based on PPI-G4-Y(30/70) or PPI-G4-Y(60/40) at different polymer/DNA mass ratios. (B) Comparison of transfection efficacies, as determined by mean fluorescence intensities of EGFP positive cells, after transfection as above. Bars represent standard deviations (SD).
**Fig. 8** **Efficacy of different PPI-Y derivatives for the transfection of siRNA.** Gene knockdown after transfection of siRNA into stably luciferase-expressing reporter cells, as indicated by the difference between luciferase activities (RLU) of cells transfected with negative control siRNA (siCtrl, black bars; normalized to 100%) and luciferase-specific siRNA (siLuc, grey bars). Complexes/nanoparticles are based on the indicated PPI dendrimers and prepared at the three different polymer/siRNA ratios 2.5, 3.75 and 5.0 (left, center and right, respectively, in each group). Knockdown efficacies were determined in (A) PC3-Luc reporter cells and (B) H441-Luc reporter cells. Bars represent standard deviations (SD).
**Fig. 9** **Efficacy of PPI-G3-Y(60/40)-based nanoparticles for delivering RNAs for the induction of base editing.** PC3 green-fluorescent reporter cells transfected with a PPI-G3-Y(60/40)-based complex/nanoparticle, containing an sgRNA + Cas-mRNA and prepared at mass ratios (A) 2.5 or (B) 3.75, for inducing a point mutation. In flow cytometry, the shift of the emission wavelength of EGFP towards blue fluorescence after this base editing event is indicated by the shift of cells from the upper left to the lower right quadrant. Original pictures of cell distribution in flow cytometry are shown, after transfection of the cells (right) vs. untransfected (UT; left), and the respective percentages of the total cell population are given.
**Fig. 10** **Efficacy of PPIY-based nanoparticles for mRNA delivery *in vivo.*** Efficacies of (A) PPI-G3-Y(60/40)- or (B) PPI-G4-Y(60/40)-based nanoparticles for mRNA delivery *in vivo* after intraperitoneal injection of mice with 20 µg or 40 µg luciferase-encoding mRNA, as indicated in the figure. Luciferase expression determined *via* live imaging of the anesthetized animals 6 h or 24 h after injection of the nanoparticles is shown.

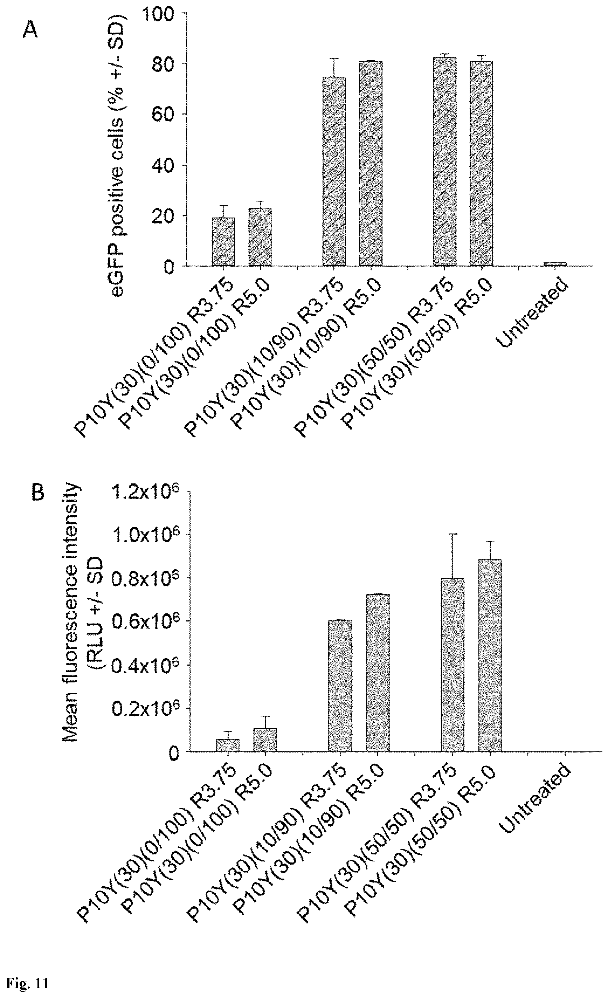

**Fig. 11** **Efficacy of different P10Y derivatives for the transfection of plasmid DNA.** (A) Percentage of EGFP-positive PC3 cells after their transfection with complexes/nanoparticles based on different P10Y derivatives at different polymer/DNA mass ratios as indicated. (B) Comparison of transfection efficacies, as determined by mean fluorescence intensities of EGFP positive cells, after transfection as above. Bars represent standard deviations (SD). P10Y(0/30), P10Y(3/27), and P10Y(15/15) correspond to P10Y(30)(0/100), P10Y(30)(10/90), and P10Y(30)(50/50), respectively (see also **Example 11).**

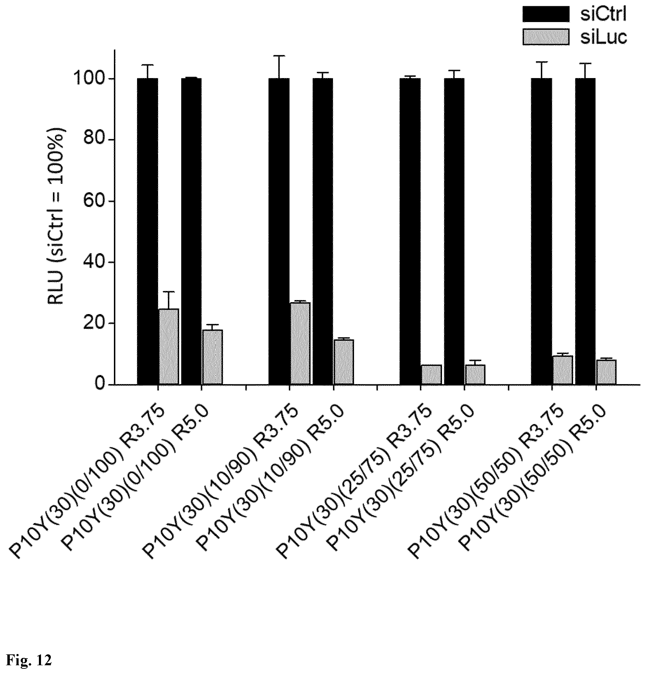

**Fig. 12** **Efficacy of different P10Y derivatives for the transfection of siRNA.** Gene knockdown after transfection of siRNA into stably luciferase-expressing reporter cells, as indicated by the difference between luciferase activities (RLU) of cells transfected with negative control siRNA (siCtrl, black bars; normalized to 100%) and luciferase-specific siRNA (siLuc, grey bars). Complexes/nanoparticles are based on the indicated P10Y derivatives and were prepared at the three different polymer/siRNA ratios 3.75 and 5.0. Bars represent standard deviations (SD). P10Y(0/30), P10Y(3/27), P10Y(8/(22/23)), and P10Y(15/15) correspond to P10Y(30)(0/100), P10Y(30)(10/90), P10Y(30)(25/75), and P10Y(30)(50/50), respectively (see also Example 11).

The term "N-Boc-tyrosine residue" refers to N-(tert.-Butoxycarbonyl)-L-tyrosin, Boc-L-tyrosin and may herein be used interchangeably with "N-Boc-tyrosine", "Boc", "Boc-Tyr", "Boc-Y", or the like. The term "Fmoc-tyrosine residue" refers to N N-(Fluorenyl-9-methoxycarbonyl)-O-tert-butyl-L-tyrosin and may herein be used interchangeably with "Fmoc-tyrosine", "Fmoc", "Fmoc-Tyr", "Fmoc-Y", or the like.

The terms "tyrosine", "tyrosine residue", "Tyr", or the respective single letter amino acid code "Y" may herein be used interchangeably and do not refer to/comprise a "N-Boc-tyrosine residue" or a "Fmoc-tyrosine residue".

In the context of the herein provided PPIs/PEIs, the present application also envisages the use of derivatives of N-Boc-tyrosine, Fmoc-tyrosine and tyrosine instead of N-Boc-tyrosine, Fmoc-tyrosine and tyrosine, respectively, however, preferred are N-Boc-tyrosine, Fmoc-tyrosine and tyrosine over their respective derivatives. Derivatives, such as derivatives of N-Boc tyrosine in the context of the present invention share similar biophysical properties with, e.g., N-Boc-tyrosine. For the avoidance of doubt and without being bound by theory, N-Boc-tyrosine or Fmoc-tyrosine are herein not to be considered derivatives of tyrosine (and vice-versa).

If not explicitly specified differently, the terms "single modified", "mono-modified", or the like in the context of the herein provided PPIs or PEIs, refers to a BOC-modified PPI or PEI. The terms "double modified", "di-modified", or the like in the context of the herein provided PPIs or PEIs, generally refers to a PPI or a PEI comprising both one or more BOC- and one or more tyrosine-modification(s).

The terms "modification", "modified" or the like in the context of the herein provided PPI or PEI refer to Boc, Fmoc, or tyrosine modifications.

The term "linked", "coupled", "fused", "bound", or the like may herein be used interchangeably. In the context of the coupling of a Boc-, a Fmoc- or a Tyr-residue to a PEI or a PPI, the coupling generally occurs *via* the free (*i.e.,* unprotected) carboxy terminus (of *e.g.,* said Boc-residue) to a terminal amino group of a PPI, a terminal amino group of a branched PEI, or an nitrogen group of said linear PEI *via* the formation of an amide bond.

The term "compound" herein refers to the herein provided PPI or to the herein provided PEI.

The terms "complex" and "nanoparticle" may herein be used interchangeably.

Concentrations, amounts, and other numerical data may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited.

The term "about" when used in connection with a numerical value is meant to encompass numerical values within a range having a lower limit that is 10% (or 5, 4, 3, 2 or 1 %) smaller than the indicated numerical value and having an upper limit that is 10% (or 5, 4, 3, 2 or 1 %) larger than the indicated numerical value.

When a numerical value is preceded by the term "about" in the context of the present invention, it is to be understood that the concrete numerical value is disclosed herein.

The term "about 0 %" herein may mean that the respective compound or feature is not present/is absent. The compound or feature may, however, be present in an amount or percentage that does not (substantially) affect the activity (e.g. the transfection efficiency of the PPI or PEI provided herein) as compared to when the compound or feature is not present/is absent.

In the foregoing detailed description of the invention, a number of individual elements, characterizing features, techniques and/or steps are disclosed. It is readily recognized that each of these has benefit not only individually when considered or used alone, but also when considered and used in combination with one another. Accordingly, to avoid exceedingly repetitious and redundant passages, this description has refrained from reiterating every possible combination and permutation. Nevertheless, whether expressly recited or not, it is understood that such combinations are entirely within the scope of the presently disclosed subject matter, unless the opposite is explicitly indicated.

All technical and scientific terms used herein, unless otherwise defined, are intended to have the same meaning as commonly understood by one of ordinary skill in the art. Reference to techniques employed herein are intended to refer to the techniques as commonly understood in the art, including variations on those techniques or substitutions of equivalent techniques that would be apparent to one of skill in the art.

All amino acid sequences provided herein are presented starting with the most N-terminal residue and ending with the most C-terminal residue, as customarily done in the art, and the one-letter or three-letter code abbreviations as used to identify amino acids throughout the present invention correspond to those commonly used for amino acids.

As used herein, the singular forms "a," "an", and "the" include the plural referents unless the context clearly indicates otherwise. The terms "include", "such as", and "the like" are intended to convey inclusion without limitation, unless otherwise specifically indicated.

As used herein, the term "or" is generally employed in its usual sense including "and/or" unless the content clearly dictates otherwise. The term "and/or" means one or all of the listed elements or a combination of any two or more of the listed elements.

As used herein, the term "comprising" also specifically includes embodiments "consisting of" and "consisting essentially of" the recited elements, unless specifically indicated otherwise.

### References

1. Noske S, Karimov M, Aigner A, Ewe A. (2020) Tyrosine-Modification of Polypropylenimine (PPI) and Polyethylenimine (PEI) Strongly Improves Efficacy of siRNA-Mediated Gene Knockdown. Nanomaterials (Basel);10.
2. Ewe A, Przybylski S, Burkhardt J, Janke A, Appelhans D, Aigner A. (2016) A novel tyrosine-modified low molecular weight polyethylenimine (P10Y) for efficient siRNA delivery in vitro and in vivo. J Control Release.;230:13-25.
3. Karimov M, et. al., (2021) Tyrosine-modified linear PEIs for highly efficacious and biocompatible siRNA delivery in vitro and in vivo. Nanomedicine: NBM;36:102403.
4. Chiper M, et. al. (2017) Self-aggregating 1.8kDa polyethylenimines with dissolution switch at endosomal acidic pH are delivery carriers for plasmid DNA, mRNA, siRNA and exon-skipping oligonucleotides. J Control Release;246:60-70.
5. Creusat G, et. al. (2012) Pyridylthiourea-grafted polyethylenimine offers an effective assistance to siRNA-mediated gene silencing in vitro and in vivo. J Control Release;157(3):418-26.
6. Creusat G, et. al. (2010) Proton sponge trick for pH-sensitive disassembly of polyethylenimine-based siRNA delivery systems. Bioconjug Chem;21(5):994-1002.
7. Creusat G & Zuber G. (2008) Self-assembling polyethylenimine derivatives mediate efficient siRNA delivery in mammalian cells. Chembiochem;9(17):2787-9.

### Examples

Certain embodiments of the invention will now be described with reference to the following examples, which are intended for the purpose of illustration only and are not intended to limit the scope of the generality hereinbefore described.

### Example 1: Materials and methods employed in Examples 2 to 12

### Example 1.A: Materials

All chemicals and reagents were of analytical grade. PPI dendrimers generation 3 and 4 (SyMO-Chem, Eindhoven, The Netherlands); branched 10 kDa polyethylenimine (branched PEI) (Polysciences, Eppelheim, Germany); linear 10 kDa polyethylenimine (linear PEI) (Sigma Aldrich, Taufkirchen, Germany), *N*-hydroxysuccinimide (NHS), dry dimethylformamide (DMF), dry dimethyl sulfoxide (DMSO) (Thermo Scientific, Schwerte, Germany); PyBOP (benzotriazol-1-yloxytripyrrolidinophosphonium hexafluorophosphate), 1-Ethyl-3-(3-dimethylaminopropyl) carbodiimide x HCl (EDCxHCl), DBU (1,8-Diazabicyclo (5.4.0) undec-7-ene), Fmoc-tyrosine and BOC-tyrosine (Carbolution Chemicals, Saarbrücken, Germany); Piperazine, NH₃ 25% in water (aq), HCl 37%, diisopropylethylamine (DIPEA), Methanol, Ethanol, Trifluoroacetic acid (TFA) (Carl Roth, Karlsruhe, Germany); Dialysis tubes (1 kDa and 3.5 kDa MWCO regenerated cellulose,) (Serva, Heidelberg, Germany).

The characterization of the polymers was performed by 1H-NMR spectroscopy (Avance III 400MHz, Bruker Corp., Billerica, MA, US). Polymers bearing completely deprotected tyrosines were dissolved in D₂O; and polymers containing BOC-tyrosine groups were dissolved in DMSO-d6 or methanol-d4.

### Example 1.B: Chemical Synthesis of Tyrosine-Modified PPI

**General method for the synthesis of PPI-Gx-Y dendrimers:** PPI dendrimers were modified by amidation of the primary amines with N-protected tyrosines. To this end, 100 mg PPI dendrimer were dissolved in 3 ml of dry dimethylformamide (DMF) in a screw cap vial and diisopropylethylamine (DIPEA) (1.6 eq, 1.5 x10⁻³, 0.264 mL) was added. The vial was flushed with nitrogen and closed. Depending on the PPI-Y derivative, Fmoc- and BOC-protected tyrosine, or only BOC-protected tyrosine (see tables below), were dissolved in 6 mL dry dimethylformamide (DMF) under stirring on a magnetic stirrer. To this solution, the coupling reagent PyBOP (benzotriazol-1-yloxytripyrrolidinophosphonium hexafluorophosphate) was added in small portions. The vial was briefly flushed with nitrogen and incubated for 5 minutes for pre-activation of the tyrosine. Afterwards, the activated tyrosine solution was added into the dendrimer-containing solution. The vial was again flushed with nitrogen and stirred at room temperature. After 24 hours, another 0.2 equivalents PyBOP were added and the reaction mixture was further stirred under inert conditions for further 2 days.

The reaction mixture was filled into a dialysis bag (1 kDa MWCO) and dialyzed against methanol (1 L) for 24 h with intermediate solvent exchanges to remove byproducts. The solution was transferred into a screw cap vial and the methanol was removed in vacuo. The modified dendrimer was dissolved in a minimum volume of methanol (1 mL), for subsequent deprotection of the tyrosine as follows:
**Fmoc deprotection:** For removing the Fmoc protection group, 4 mL of a reagent mixture containing 4% (v/v) DBU (1,8-Diazabicyclo (5.4.0) undec-7-ene), 10% (w/v) piperazine, 10% (v/v) NH₃ (aq) in DMF/ethanol (80/20 v/v) was added and stirred overnight. During the de-protection process, a white precipitate of the dibenzofulvene group was formed. The suspension was transferred into a centrifuge tube and centrifuged for 15 minutes at 4000 rpm in order to remove the major parts of the side-product. Next, the cloudy solution was transferred into a dialysis bag (1 kDa MWCO) and dialyzed against methanol for 2 days and 70% ethanol for 1 day to further remove impurities. The solvent of the clear, yellowish solution was removed in vacuo until a white dispersion appeared (precipitation of the water-insoluble product). A small amount of deionized water (2 mL) was added and the dispersion was frozen and lyophilized. This yielded a white/yellowish powder of dendrimer with partially deprotected tyrosine-modified PPI dendrimers.

**BOC deprotection:** For the deprotection of the BOC-tyrosine modified PPI dendrimers, an excess of the trifluoroacetic acid (TFA) (~ 10 mL) was slowly added. An immediate gas formation was observed and the reaction mixture was stirred overnight for completion. Next, 20 mL ethanol were added and the solvents were removed in a rotary evaporator. This step was repeated two times in order to remove TFA residuals. The crude product was dissolved in methanol and transferred into a dialysis bag (1 kDa MWCO) and dialyzed against 1 L methanol for 24 h, 1 L 0.1 M HCl for 24 h and 2 L deionized water for 48 h) with regular solvent exchanges. Subsequently, the solution was frozen and lyophilized. The completely deprotected tyrosine-modified PPI dendrimers were obtained as white, yellowish powders.

**Completely BOC-protected tyrosine-modified PPI dendrimers:** In case all amines were intended to remain modified with Boc-protected tyrosines (PPI-G4-100/0), the crude reaction mixture was extensively dialyzed (1 kDa MWCO) against methanol (1 L) for 48 h and 70% ethanol (1 L) for 24 h. The solvent was removed in vacuo until a white precipitate appeared. To the dispersion, 2 mL deionized water was added, then frozen and lyophilized.

### Calculation of the reaction conditions for herein employed PPI-tyrosine derivatives:

| PPI | M (g/mol) | M (g) | n (mol) | # NH₂ groups | n (NH₂ groups) | eq |
|---|---|---|---|---|---|---|
| Generation 3 | 1686.8 | 0.1 | 5.93 x10⁻⁵ | 16 | 0.95 x10⁻³ | 1 |
| Generation 4 | 3514 | 0.1 | 2.85 x10⁻⁵ | 32 | 0.91 x10⁻³ | 1 |

| **PPI-G3-(0/100)** | M (g/mol) | eq | n (mol) | m (g) |
|---|---|---|---|---|
| BOC-tyrosine | 281.3 | 1.1 | 1.04 x10⁻³ | 0.294 |
| PyBOP 1. portion | 520.39 | 1.4 | 1.33 x10⁻³ | 0.691 |
| PyBOP 2. portion | 520.39 | 0.2 | 0.19 x10⁻³ | 0.01 |
| DIPEA | 129.25 | 1.6 | 1.5 x10⁻³ | 0.2 (0.265 mL) |

| **PPI-G3-(60/0)** | M (g/mol) | eq | n (mol) | m (g) |
|---|---|---|---|---|
| BOC-tyrosine | 281.3 | 0.65 | 0.616 x10⁻³ | 0.173 |
| PyBOP 1. portion | 520.39 | 0.8 | 0.76 x10⁻³ | 0.395 |
| PyBOP 2. portion | 520.39 | 0.2 | 0.19 x10⁻³ | 0.01 |
| DIPEA | 129.25 | 1.6 | 1.5 x10⁻³ | 0.2 (0.265 mL) |

| **PPI-G3-(60/40)** | M (g/mol) | eq | n (mol) | m (g) |
|---|---|---|---|---|
| Tyrosine (Total) | | 1.1 | 1.04 x10⁻³ | |
| BOC-tyrosine | 281.3 | 0.66 | 0.626 x10⁻³ | 0.176 |
| Fmoc-tyrosine | 403.43 | 0.44 | 0.417 x10⁻³ | 0.168 |
| PyBOP 1. portion | 520.39 | 1.4 | 1.33 x10⁻³ | 0.691 |
| PyBOP 2. portion | 520.39 | 0.2 | 0.19 x10⁻³ | 0.01 |
| DIPEA | 129.25 | 1.6 | 1.5 x10⁻³ | 0.2 (0.265 mL) |

| **PPI-G4-(0/100) and PPI-G4-(100/0)** | M (g/mol) | eq | n (mol) | m (g) |
|---|---|---|---|---|
| BOC-tyrosine | 281.3 | 1.1 | 1.0 x10⁻³ | 0.282 |
| PyBOP 1. portion | 520.39 | 1.4 | 1.27 x10⁻³ | 0.663 |
| PyBOP 2. portion | 520.39 | 0.2 | 0.18 x10⁻³ | 0.01 |
| DIPEA | 129.25 | 1.6 | 1.45 x10⁻³ | 0.19 (0.255 mL) |

| **PPI-G4-(30/0)** | M (g/mol) | eq | n (mol) | m (g) |
|---|---|---|---|---|
| BOC-tyrosine | 281.3 | 0.35 | 0.32 x10⁻³ | 0.09 |
| PyBOP 1. portion | 520.39 | 0.5 | 0.46 x10⁻³ | 0.237 |
| PyBOP 2. portion | 520.39 | 0.2 | 0.18 x10⁻³ | 0.01 |
| DIPEA | 129.25 | 1.6 | 1.45 x10⁻³ | 0.19 (0.255 mL) |

| **PPI-G4-(60/0)** | M (g/mol) | eq | n (mol) | m (g) |
|---|---|---|---|---|
| BOC-tyrosine | 281.3 | 0.65 | 0.6 x10⁻³ | 0.167 |
| PyBOP 1. portion | 520.39 | 0.8 | 0.73 x10⁻³ | 0.379 |
| PyBOP 2. portion | 520.39 | 0.2 | 0.18 x10⁻³ | 0.01 |
| DIPEA | 129.25 | 1.6 | 1.45 x10⁻³ | 0.19 (0.255 mL) |

| **PPI-G4-(60/40)** | M (g/mol) | eq | n (mol) | m (g) |
|---|---|---|---|---|
| Tyrosine (Total) | | 1.1 | 1.0 x10⁻³ | |
| BOC-tyrosine | 281.3 | 0.66 | 0.6 x10⁻³ | 0.169 |
| Fmoc-tyrosine | 403.43 | 0.44 | 0.4 x10⁻³ | 0.162 |
| PyBOP 1. portion | 520.39 | 1.4 | 1.27 x10⁻³ | 0.663 |
| PyBOP 2. portion | 520.39 | 0.2 | 0.18 x10⁻³ | 0.01 |
| DIPEA | 129.25 | 1.6 | 1.45 x10⁻³ | 0.19 (0.255 mL) |

| **PPI-G4-(30/70)** | M (g/mol) | eq | n (mol) | m (g) |
|---|---|---|---|---|
| Tyrosine (Total) | | 1.1 | 1.0 x10⁻³ | |
| BOC-tyrosine | 281.3 | 0.33 | 0.3 x10⁻³ | 0.085 |
| Fmoc-tyrosine | 403.43 | 0.77 | 0.7 x10⁻³ | 0.283 |
| PyBOP 1. portion | 520.39 | 1.4 | 1.27 x10⁻³ | 0.663 |
| PyBOP 2. portion | 520.39 | 0.2 | 0.18 x10⁻³ | 0.01 |
| DIPEA | 129.25 | 1.6 | 1.45 x10⁻³ | 0.19 (0.255 mL) |

**Synthesis of tyrosine-modified branched polyethylenimine P10Y(30)(0/100):** In a screw cap vial, branched 10 kDa PEI (0.1 g, 2.32 x10⁻³ mol ethylenimine monomer, 1eq) was dissolved in 2 mL dry dimethyl sulfoxide (DMSO) and diisopropylethylamine (DIPEA) (1.5 eq, 3.5 x10⁻³ mol, 0.61 mL) was added. The vial was flushed with nitrogen and closed. In a separate vial, BOC-protected tyrosine (0.35 eq, 0.81 x10⁻³ mol, 0.229 g), *N*-hydroxysuccinimide (NHS) (0.46 eq, 1.05 x10⁻³ mol, 0.122 g) were dissolved in 3 mL dry DMSO. To this solution, 1-Ethyl-3-(3-dimethylaminopropyl) carbodiimide x HCl (EDCxHCl) (0.46 eq, 1.05 x10⁻³ mol, 0.203 g) was added. The vial was flushed with nitrogen and stirred for 30 minutes for pre-activation. Next, the tyrosine-containing solution was added dropwise into the PEI solution and stirred under inert conditions at room temperature. After 24 h, another portion of EDCxHCl (0.1 eq, 0.2 x10⁻³ mol, 0.04 g) was added to the reaction mixture and stirred for another 48 h. Next, the reaction mixture was purified by dialysis (3.5 kDa MWCO) against methanol (1 L) for 24h. The solution was transferred into a screw cap vial and the solvent was removed in vacuo. The polymer was dissolved in a minimum amount of methanol (1 mL) and an excess of trifluoroacetic acid (8 mL) was added for BOC deprotection. After 24 h stirring at room temperature, TFA was removed in vacuo by co-evaporation with ethanol (2 x 20 mL). Finally, the polymer was dissolved in methanol (5 mL) and further purified by dialysis (3.5 kDa MWCO) against methanol (1 L) for 24 h, 0.1 M HCl (1 L) for 24 h and deionized water (2L) for 48 h with intermediate solvent exchanges. The purified polymer (P10Y(30)(0/100)) was frozen and lyophilized to obtain a white, slightly yellowish fluffy powder.

**Synthesis of tyrosine-modified branched polyethylenimine containing BOC-Tyr groups (P10Y(30)(xx**ᵣₑₗ**/zz**ᵣₑₗ**)):** Similarly as described above, branched 10 kDa PEI (0.1 g, 2.32 x10⁻³ mol ethylenimine monomer, 1eq) was dissolved in 2 mL dry DMSO and DIPEA (1.5 eq, 3.5 x10⁻³ mol, 0.61 mL) was added. In a second vial, BOC-tyrosine and Fmoc-tyrosine in the desired ratio, as listed below, were dissolved in 3 mL dry DMSO and PyBOP (0.46 eq, 1.06 x10⁻³ mol, 0.55g) was added. The solution was stirred for 5 minutes for pre-activation and then transferred to the PEI-containing solution. The reaction mixture was stirred under inert conditions and after 24 h, a second portion of PyBOP (0.1 eq, 0.23 x10⁻³ mol, 0.119 g) was added. After further 48 h stirring at room temperature, the reaction mixture was dialyzed (3.5 kDa MWCO) against methanol (1 L) for 24 h with intermediate solvent exchange. The solvent was removed in vacuo and the crude polymer was dissolved in a minimum amount of methanol (1 mL). For Fmoc deprotection, 4 mL of a reagent mixture containing 4% (v/v) DBU (1,8-Diazabicyclo (5.4.0) undec-7-ene), 10% (w/v) piperazine, 10% (v/v) NH₃ (aq) in DMF/ethanol (80/20 v/v) was added and stirred overnight. The reaction mixture was finally purified by dialysis (3.5 kDa MWCO) against methanol (1 L) for 48 h and 70% ethanol (1 L) for 24h. The solvent was removed in vacuo and the polymer was dispersed in deionized water, frozen and lyophilized.

| P10Y(30(10/90)); xx=3, zz=27 | M (g/mol) | eq | n (mol) | m (g) |
|---|---|---|---|---|
| Modifications (Total) | | 0.35 | 0.81 x10⁻³ | |
| BOC-tyrosine | 281.3 | 0.035 | 0.081 x10⁻³ | 0.023 |
| Fmoc-tyrosine | 403.43 | 0.315 | 0.731 x10⁻³ | 0.295 |
| PyBOP 1. portion | 520.39 | 0.46 | 1.06 x10⁻³ | 0.55 |
| PyBOP 2. portion | 520.39 | 0.1 | 0.23 x10⁻³ | 0.119 |
| DIPEA | 129.25 | 1.5 | 3.5 x10⁻³ | 0.45 (0.6 mL) |

| P10Y(30(25/75)); xx=7.5, zz=22.5 | M (g/mol) | eq | n (mol) | m (g) |
|---|---|---|---|---|
| Modifications (Total) | | 0.35 | 0.81 x10⁻³ | |
| BOC-tyrosine | 281.3 | 0.086 | 0.2 x10⁻³ | 0.057 |
| Fmoc-tyrosine | 403.43 | 0.263 | 0.609 x10⁻³ | 0.246 |
| PyBOP 1. portion | 520.39 | 0.46 | 1.06 x10⁻³ | 0.55 |
| PyBOP 2. portion | 520.39 | 0.1 | 0.23 x10⁻³ | 0.119 |
| DIPEA | 129.25 | 1.5 | 3.5 x10⁻³ | 0.45 (0.6 mL) |

| P10Y(30(50/50)); xx=15, zz=15 | M (g/mol) | eq | n (mol) | m (g) |
|---|---|---|---|---|
| Modifications (Total) | | 0.35 | 0.81 x10⁻³ | |
| BOC-tyrosine | 281.3 | 0.175 | 0.406 x10⁻³ | 0.114 |
| Fmoc-tyrosine | 403.43 | 0.175 | 0.406 x10⁻³ | 0.164 |
| PyBOP 1. portion | 520.39 | 0.46 | 1.06 x10⁻³ | 0.55 |
| PyBOP 2. portion | 520.39 | 0.1 | 0.23 x10⁻³ | 0.119 |
| DIPEA | 129.25 | 1.5 | 3.5 x10⁻³ | 0.45 (0.6 mL) |

**Synthesis of tyrosine-modified linear polyethylenimine LP10Y(35)(0/100):** In a screw cap vial, 100 mg linear 10 kDa PEI (2.32 x10⁻³ mol ethylenimine monomer, 1eq) was dissolved in 3 mL dry DMF and heated to 70°C using a water bath. After complete dissolution of the polymer, DIPEA (1.5 eq, 3.5 x10⁻³ mol, 0.61 mL) was added. In a separate vial, BOC-tyrosine (0.4 eq, 0.93 x 10⁻³ mol, 0.261 mg) was dissolved in 4 mL dry DMF and PyBOP (0.46 eq, 1.06 x10⁻³ mol, 0.55g) was added and stirred for 5 min at room temperature. After the short pre-activation, the tyrosine-containing solution was slowly added into the PEI solution and stirred under inert conditions at room temperature. After 24 h, a second portion of PyBOP (0.1 eq, 0.23 x10⁻³ mol, 0.119 g) was added and stirred for further 48 h. Next, the reaction mixture was dialyzed (3.5 kDa MWCO) against methanol (1L) for 24h. The organic solvent was removed in vacuo and the crude polymer was dissolved in a minimum amount of methanol (1 mL). To the polymer solution, 5 mL TFA was added for BOC deprotection and stirred overnight. The TFA was removed in vacuo by co-evaporation with ethanol (2 x 20 mL). Finally, the polymer was dissolved in methanol (5 mL) and further purified by dialysis (3.5 kDa MWCO) against methanol (1 L) for 24 h, 0.1 M HCl (1 L) for 24 h and deionized water (2L) for 48 h with intermediate solvent exchanges. The purified polymer (LP10Y(35)(0/100)) was frozen and lyophilized to obtain white, fluffy powder.

**Synthesis of tyrosine-modified linear polyethylenimine comprising BOC groups LP10Y(35)(xx**ᵣₑₗ**/zz**ᵣₑₗ**):** First, 10 kDa linear PEI (0.1 g, 2.32 x10⁻³ mol ethylenimine monomer, 1eq) was dissolved in 3 mL dry DMF and heated to 70 °C for dissolution, prior to adding DIPEA (1.5 eq, 3.5 x10⁻³ mol, 0.61 mL). In a second vial, BOC-tyrosine and Fmoc-tyrosine in the desired ratio, as listed below, were dissolved in 4 mL dry DMF and PyBOP was added. The solution was stirred for 5 minutes for pre-activation and then transferred to the PEI-containing solution. The reaction mixture was stirred under inert conditions at room temperature. After 24 h, a second portion of PyBOP (0.1 eq, 0.23 x10⁻³ mol, 0.119 g) was added. After further 48 h stirring at room temperature, the reaction mixture was dialyzed (3.5 kDa MWCO) against methanol (1 L) for 24 h with intermediate solvent exchange. The solvent was removed in vacuo and the crude polymer was dissolved in a minimum amount of methanol (1 mL). For Fmoc deprotection, 4 mL of a reagent mixture containing 4% (v/v) DBU (1,8-Diazabicyclo (5.4.0) undec-7-ene), 10% (w/v) piperazine, 10% (v/v) NH₃ (aq) in DMF/ethanol (80/20 v/v) was added and stirred overnight. The reaction mixture was finally purified by dialysis (3.5 kDa MWCO) against methanol (1 L) for 48 h and 70% ethanol (1 L) for 24h. The solvent was removed in vacuo and the polymer was dispersed in deionized water, frozen and lyophilized.

| LP10Y(35(50/50)); xx=18, zz=18 | M (g/mol) | eq | n (mol) | m (g) |
|---|---|---|---|---|
| Modifications (Total) | | 0.4 | 0.929 x10⁻³ | |
| BOC-tyrosine | 281.3 | 0.2 | 0.464 x10⁻³ | 0.131 |
| Fmoc-tyrosine | 403.43 | 0.2 | 0.464 x10⁻³ | 0.187 |
| PyBOP 1. portion | 520.39 | 0.46 | 1.06 x10⁻³ | 0.55 |
| PyBOP 2. portion | 520.39 | 0.1 | 0.23 x10⁻³ | 0.119 |
| DIPEA | 129.25 | 1.5 | 3.5 x10⁻³ | 0.45 (0.6 mL) |

| LP10Y(20(100/0)); xx=20, zz=0 | M (g/mol) | eq | n (mol) | m (g) |
|---|---|---|---|---|
| Modifications (Total) | | 0.25 | 0.58 x10⁻³ | |
| BOC-tyrosine | 281.3 | 0.035 | 0.58 x10⁻³ | 0.163 |
| Fmoc-tyrosine | 403.43 | | | |
| PyBOP 1. portion | 520.39 | 0.3 | 0.7 x10⁻³ | 0.362 |
| PyBOP 2. portion | 520.39 | 0.05 | 0.12 x10⁻³ | 0.06 |
| DIPEA | 129.25 | 1.5 | 3.5 x10⁻³ | 0.45 (0.6 mL) |

| LP10Y(10(100/0)); xx=10, zz=0 | M (g/mol) | eq | n (mol) | m (g) |
|---|---|---|---|---|
| Modifications (Total) | | 0.15 | 0.35 x10⁻³ | |
| BOC-tyrosine | 281.3 | 0.15 | 0.35 x10⁻³ | 0.098 |
| Fmoc-tyrosine | 403.43 | | | |
| PyBOP 1. portion | 520.39 | 0.2 | 0.464 x10⁻³ | 0.24 |
| PyBOP 2. portion | 520.39 | 0.05 | 0.12 x10⁻³ | 0.06 |
| DIPEA | 129.25 | 1.5 | 3.5 x10⁻³ | 0.45 (0.6 mL) |

### Example 1.C: Nucleic acid preparation

*In* ***Vitro* mRNA Transcription:** To generate capped and poly(A) tailed mRNA encoding the sequence for the Luciferase- or eGFP-gene, the following IVT vectors were used:
pTK305 (encoding firefly luciferase) was a gift from Ron Weiss (Addgene plasmid # 66812 ; http://n2t.net/addgene:66812 ; RRID:Addgene_66812),
pRNA2-(A)128 (encoding eGFP) was a gift from Stephen Ikeda (Addgene plasmid # 174006 ; http://n2t.net/addgene:174006 ; RRID:Addgene_174006),
pT7-VEE-GFP (encoding eGFP and noninfectious (non-packaging), self-replicating Venezuelan equine encephalitis (VEE) virus RNA replicon) was a gift from Steven Dowdy (Addgene plasmid # 58977 ; http://n2t.net/addgene:58977 ; RRID:Addgene_58977).

For efficient translation and increased capping efficiency, the CleanCap^{®} was used. For this, the T7 promoter sequence needs to be followed by an AG initiation sequence on the DNA template. This was done by site-directed mutagenesis. After efficient insertion of the additional Nucleotide, plasmids were transformed into DH5a (Thermo Fisher Scientific Inc., Waltham, Massachusetts, USA) or NH5a (New England Biolabs, Ipswich, MA, USA), purified with NucleoBond^{®} Mini or NucleoBond^{®} Xtra Midi/Maxi Kit (Macherey-Nagel, Düren, Germany) and sequenced using SangerSequencing.

For IVT, plasmid templates were linearized with either SacII-, DraIII, PvuII or MluI endonucleases (New England Biolabs, Ipswich, MA, USA) and purified using Roti-Phenol/Chloroform extraction. IVT mRNAs were generated according to the manufacturer's guideline using 1 µg linearized and purified template DNA and HiScribe T7 CleanCap^{®} mRNA Kit (New England Biolabs, Ipswich, MA, USA). MRNA containing a poly(A) tail (sequence encoded on plasmid pRNA2-(A)-128 were immediately purified using Monarch RNA Cleanup Kit (New England Biolabs, Ipswich, MA, USA). Otherwise, the *E.coli* poly(A) polymerase (New England Biolabs, Ipswich, MA, USA) was used for catalyzing the addition of AMP from ATP to the 3' end of the RNA for 30 min at 37°C, prior to purification as above.

| **siRNA** | **Sequence (5' - 3')** | | **Vendor** | **SEQ ID NO:** |
|---|---|---|---|---|
| siLuc 2 | sense | CGUACGCGGAAUACUUCGA dTdT | Dharmacon, Lafayette, CO, USA | 1 |
| | antisense | UCGAAGUAUUCCGCGUACG dTdT | | 2 |
| siLuc 3 | sense | CUUACGCUGAGUACUUCGA dTdT | Eurogentec, Seraing, Belgium | 3 |
| | antisense | UCGAAGUACUCAGCGUAAG dTdT | | 4 |
| siEGF P | sense | GCAGCACGACUUCUUCAAG dTdT | Eurogentec , Seraing, Belgium | 5 |
| | antisense | CUUGAAGAAGUCGUGCUGC dTdT | | 6 |

In the above table, dTdT' stands for a deoxythymidine dinucleotide overhang.

**Plasmid DNA:** Plasmid DNA (pDNA) encoding the sequence for eGFP expression in mammalian cells (peGFP-NA, Clontech) was transformed in *E.coli,* strain DH5a, grown in LB-medium (5g yeast extract, 10g NaCl, 10g tryptone, 1 L dH₂O) and purified after cultivation using the NucleoBond^{®} Xtra Midi/Maxi Kit (Macherey-Nagel, Düren, Germany) according to the manufacturer's protocol. The pDNA was dissolved in sterile dH₂O, the concentration was determined using a Nanodrop^{®}2000 (ThermoFisher, Schwerte, Germany) and pDNA was stored at -20°C.

**MiniCircle:** The Minicircle was produced from a parental-plasmid DNA containing the eGFP reporter sequence using the MC-Easy^{™} Kit (System Biosciences (SBI) Palo Alto, USA) according to the manufacturer's protocol. The ZYCY10P3S2T *E.coli* Minicircle producer strain was used, which stably expresses the set of arabinose-inducible Minicircle-assembly enzymes ϕC31 integrase and I-SceI endonuclease simultaneously. After incubation with arabinose-containing induction media, the ϕC31 integrase generates Minicircle DNA by recombination of sequences containing two recognition attachment sites (att) at the full-size parental plasmid. Remaining fragments from the plasmid backbone were degraded by SceI endonuclease. The produced Minicircle DNA was purified using the NucleoBond^{®} Xtra Midi/Maxi Kit (Macherey-Nagel, Düren, Germany) according to the manufacturer's protocol.

**Base Editing:** The mRNA Cas9-ABE3030 (containing modified mCTP and mψUTP) as well as the sequence of the sgRNA (5'- GCCGTAGGTCAGGGTGGTCACG -3') for the conversion of GFP to BFP was used, according to Schmitt et al. Genome Biology (2023) 24:254*.*

### Example 1.D: Cell lines and cell culture

Cell culture media were purchased from Sigma-Aldrich (Taufkirchen, Germany) and fetal calf serum was from Serana (Pessin, Germany). All cell culture plastics and consumables were from Sarstedt (Nümbrecht, Germany). The cell lines PC3 (prostate carcinoma) and H441 (lung adenocarcinoma) were obtained from ATCC/LGC Promochem (Wesel, Germany). Cells were routinely tested for Mycoplasma using the proposed protocol of the collection of methods of the German Federal Working Group on Genetic Engineering with the MB Taq Polymerase (Minerva Biolabs, Berlin, Germany) or the Venor^{®} GeM Classic Kit (Minerva Biolabs, Berlin, Germany) and determined to be free of any contamination.

The cells were grown in Iscove's Modified Dulbecoo Medium (IMDM), supplemented with 10%FCS and 2 mM alanyl-glutamine, in absence of antibiotics and cultivated in a humid atmosphere at 37°C and 5% CO₂. The Reporter cell lines (PC3-Luc and H441-Luc) were stably transduced by lentiviral transduction. The luciferase plasmid pLenti PGK-V5-LUC Puro (w543-1) was a gift from Eric Campeau and Paul Kaufman (University of Massachusetts, Worchester, MA, USA; Addgene plasmid # 19360). Reporter cell lines were cultivated in the above-mentioned media and selected by using purmoycin.

### Example 1.E: Complex preparation and transfection

Polymer/nucleic acid complexes were prepared at a mass ratio of 2.5, unless indicated otherwise. Complexation involves dilution of 0.5 µg nucleic acid (for example mRNA) in 25 µl HN buffer (150 mM NaCl, 10 mM HEPES), mixed by pipetting up and down with 1.25 µg polymer diluted in the same volume and buffer and allowed to incubate for ~30 min at room temperature.

For the different transfections, the respective cells were seeded at least 24h prior transfection as follows:

| **well format** | **final volume** | **mRNA transfection** | **siRNA or DNA transfection** |
|---|---|---|---|
| 24 well | 500 µl | 50,000 cells/well | 35,000 cells/well |
| 48 well | 250 µl | 25,000 cells/well | 20,000 cells/well |
| 96 well | 100 µl | 12,500 cells/well | 10,000 cells/well |

The complexes were then added to the pre-seeded cells and incubated for 24 - 72h.

### Example 1.F: Analysis of cytotoxicity and viability

Determination of cytotoxicity and viability included proliferation assays as well as acute lactate dehydrogenase release, as a marker for acute cell damage caused by the polyplexes. The commercially available mRNA GFP (5mou) (Trilink, San Diego, USA) was used for complexation. The LDH release assay was carried out using the Cytotoxicity Detection Kit (Roche, Mannheim, Germany) according to the manufacturer's protocol. 24 h after transfection of the cells, the conditioned media was collected. As a positive control for maximum LDH release, cells were treated with Triton-X100 at a final concentration of 2% for at least 20 min, prior to collecting the media. Medium from untransfected cells served as negative control, and fresh medium served as blank value correction. The enzymatic reaction of this colorimetric assay was based on the LDH released from damaged cells into the medium and was initiated by mixing 50 µl media of the treated samples with 50 µl reaction mix (catalyst solution: Diaphorase/NAD+ mixture and dye solution: INT and sodium lactate). After 30 min incubation in the dark, the reaction was stopped using 50 µl of 1 M acetic acid and the absorbance at 490/620 nm was measured using a plate reader.

### Example 1. G: Readouts for the analysis of transfection efficacy

**Luciferase activity:** The luciferase activity after gain-of-function (luciferase mRNA) transfection was determined after 24 h, while knockdown efficiencies after siRNA transfection were determined after 72 h. For this, the Beetle-Juice Kit (PJK, Kleinblittersdorf, Germany) was used. The medium was aspirated and the cells were lysed with 100 µl cold Luciferase Cell Culture Lyse Reagent (Promega, Mannheim, Germany) for 30 min at RT under continuous gentle shaking. 10 µl lysate was mixed with 25 µl substrate and the luminescence was measured immediately in a luminometer (Berthold, Bad Wildbad, Germany)

**Incucyte imaging:** For real-time cell monitoring, surveillance of cell health and viability by visualizing cell morphology and confluence as well as determination of transfection efficiency and reporter gene expression, the Incucyte^{®} instrument (Sartorius, Göttingen, Germany) was used. The Live-Cell Analysis System is positioned in an incubator with an humid atmosphere at 37°C and 5% CO₂. For standard transfection experiments, the cells were seeded in well-plates and transfected with the polyplexes. The well-plate was then placed in the holder of the Incucyte^{®} instrument and images were taken automatically at defined time intervals for a certain period of time. Confluence and fluorescence intensities were determined using the manufacturer's software.

**Flow cytometry:** For determination of fluorescent reporter gene expression, an acoustic flow cytometry instrument Attune^{®} or AttuneNxt^{®} (ThermoFisher, Schwerte, Germany; Life Technologies, Darmstadt, Germany) was used. 24 h after mRNA transfection, or 72 h after siRNA/DNA transfection, the cells were trypsinized, washed with PBS and centrifuged (5 min, 1000g), prior to resuspension in 500 µl PBS containing 1% BSA. At least 10,000 viable and single cells were gated and analyzed using the manufacturer's software.

### Example 1.H: In vivo analysis of mRNA expression

Immunodeficient mice (NSG mice) were kept at 25 °C in a humidified atmosphere, 12 h light/dark cycle with food and water ad libitum. All animal studies were approved by the local authorities (Landesdirektion Sachsen).

For *in vivo* analysis of mRNA expression, 20 µg luciferase mRNA (1 mg/mL) was diluted in 75 µL complexation buffer. In a separate vial, 50 µg of PPI-G3-Y (60/40) or PPI-G4-Y (60/40) (mass ratio 2.5) was diluted in 75 µLcomplexation buffer. The mRNA solution was added to the dendrimer solution and rapidly mixed by vigorous pipetting, prior to incubating for 30 minutes at room temperature. 150 µL (20 µg) or 300 µL(40 µg) were intraperitoneally injected into mice.

After 6 h and 24 h, luciferase expression was evaluated by live animal bioluminescence imaging using the IVIS *in vivo* imaging system (Perkin Elmer, MA, USA). Mice were anesthetized under isoflurane and 150 µLLuciferin (30 mg/mL) was intraperitoneally injected. After 5 minutes, mice were imaged over a period of 20 minutes. The images were analyzed using the Living Image analysis software (Perkin Elmer, MA, USA).

### Example 2: Surprising identification of N-Boc-tyrosine-modified PPIs and their advantageous effects on transfection efficiencies

Previous studies in the context of the tyrosine-modification of PPIs relied on the use of BOC-protected tyrosine, with subsequent de-protection for yielding tyrosine-modified polymers. However, for the first time, the present inventors accidentally employed PPIs comprising non-deprotected BOC-residues and surprisingly found that such PPI derivatives showed enhanced transfection efficacy over their deprotected counterparts. This indicated that the presence of BOC groups might exert beneficial effects on the biological efficacies of the nanoparticles based on PPI polymers. Consequently, based on this unexpected finding, polymers were synthesized where BOC-Tyr modifications alone or both modifications, *i.e.,* Tyr and BOC-Tyr, in combination were systematically implemented at defined ratios. For this, the present inventors developed an orthogonal protection strategy employing BOC-tyrosine and Fmoc-tyrosine, allowing the specific cleavage of the Fmoc group while retaining the other modification in its BOC-Tyr form (see **Example 1.B).** This allowed for precisely fine-tuning the desired percentages of the modifications. Based on this reaction scheme, the present inventors synthesized a range of different single and double-modified PPIs and PEIs comprising BOC-Tyr and/or Tyr derivatives. When using the generation 4 PPI (PPI-G4) as starting material, this yielded the PPI-G4 structures shown in **Formula II,** with R representing BOC-Tyr and/or Tyr modification of the primary amines of PPI at a ratio defined by percentages: PPI-G4-Y(xx/zz); xx = absolute/total percentage of BOC-tyrosine modification and zz = absolute/total percentage of tyrosine modification, related to maximum possible degree of modification of PPI-G4. Three PPI-G4 derivatives with full (PPI-G4-Y(100/0)) or partial (PPI-G4-Y(30/0), PPI-G4-Y(60/0)) mono-substitution of primary amines with BOC-Tyr and two PPIs 100% modified with a combination of BOC-Tyr and Tyr at defined ratios (PPI-G4-Y(60/40), PPI-G4-Y(30/70)) were synthesized and compared to the previously described [1] PPI-Y, now termed PPI-G4-Y(0/100) with 100% Tyr modification.

| **BOC-Tyr** | **BOC-Tyr & Tyr** | **Tyr** |
|---|---|---|
| PPI-G4-Y(100/0) | PPI-G4-Y(60/40) | PPI-G4-Y(0/100) |
| PPI-G4-Y(30/0) | PPI-G4-Y(30/70) | |
| PPI-G4-Y(60/0) | | |

### Example 3: Efficacy of double-modified PPI-G4 derivatives for mRNA transfection

The efficacy of different chemically modified PPI derivatives for mRNA transfection was tested in PC3 prostate carcinoma cells and compared to PPIY-G4-(0/100), as well as to the commercially available jetMessenger^{®} (JM) transfection reagent, here serving as a gold standard. An mRNA encoding for Enhanced Green Fluorescent Protein (EGFP) was employed and EGFP expression was determined in an Incucyte instrument over a time period of 22 h. For complex formation, three polymer/mRNA mass ratios (2.5, 3.75 and 5.0) were used, with 1 µg mRNA per 24 well. Very little EGFP expression as determined by total integrated fluorescence intensity, was obtained after transfection of mRNA complexed with PPI-G4-Y(0/100) or when using JM **(****Fig. 1A****).**

When switching to PPI derivatives mono-modified only with BOC-Tyr, a low degree 30% or 60% primary amine modification resulted in some EGFP expression, but only at the highest polymer/mRNA ratio (3.75) and with efficacies below jetMessenger^{®} (data not shown). Dendrimers with 100 % BOC-Tyr modification were associated with low solubility (leading to precipitate formation) and were thus not pursued further in this context. Notably, however, the combination of both modifications (BOC-Tyr and Tyr) with a 100 % total degree of primary amine substitution yielded a substantial increase in mRNA transfection efficacy. This was particularly true for the PPI-G4-Y(60/40) derivative, with total integrated EGFP fluorescence intensities substantially above the JM or PPI-G4-Y(0/100)-based transfections **(****Fig. 1A****).** In the case of this dendrimer, transfection efficacies were further improved with increasing polymer/mRNA ratio, with mass ratio 5 yielding best results. The time-dependent monitoring of EGFP expression revealed a very rapid onset, with fluorescence already detectable within the first 2 h. Notably, decreasing the BOC-Tyr modification over the Tyr modification led to lower EGFP expression levels and a delayed onset of EGFP fluorescence after mRNA transfection, as seen in the case of PPI-G4-Y(30/70) dendrimer **(****Fig. 2A****).** Despite lower transfection efficacies, a dependence on the polymer/mRNA mass ratio was again observed, with mass ratios 3.75 and 5.0 being above JM and PPI-G4-Y(0/100). Taken together, this indicates the BOC-Tyr modification and in particular the BOC-Tyr + Tyr combination as key modification of PPI-G4 dendrimers for mRNA transfection and identifies a 60/40 ratio, with a slight excess of BOC-Tyr- over Tyr-modified primary amines, as particularly efficient.

### Example 4: Double-modified PPI-G3 derivatives for mRNA transfection

Next, the present inventors aimed at reducing the polymer size by switching to 3^{rd} generation PPI-G3 dendrimers. Based on the above results, the present inventors focused on the BOC-Tyr & Tyr double modification at the 60/40 ratio (PPI-G3-Y(60/40)) and its BOC-Tyr mono-substituted counterpart, PPI-G3-Y(60/0), and compared it to PPI-G3-Y(0/100) without BOC-Tyr modification. As seen before in the case of PPI-G4, the unmodified PPI-G3 dendrimer was inefficacious (data not shown). The Tyr mono-modification (PPI-G3-Y(0/100) showed little mRNA transfection efficacy, comparable to JM **(****Fig. 3A****).** In contrast, the PPI-G3 dendrimer already benefitted from 60% BOC-Tyr grafting, with EGFP expression levels as determined by total integrated fluorescence intensity being well above those in JM- or PPI-G3-Y(0/100)-transfected cells. This was obtained at polymer/mRNA mass ratios 3.75 or 5.0 **(****Fig. 3A****).** As seen before in the case of PPI-G4 dendrimers, however, highest EGFP expression upon mRNA transfection was obtained in the case of the BOC-Tyr & Tyr 60/40 double modification, with PPI-G3-Y(60/40) yielding total integrated EGFP fluorescence intensities > 5-fold above JM or PPI-G3-Y(0/100) **(****Fig. 4A****).** Comparable to its 4^{th} generation counterpart, a rapid onset of EGFP positivity after ~ 2 h was observed. All tested mass ratios used in mRNA complexation resulted in much improved transfection efficiencies as compared to the both tested controls, with ratio 3.75 yielding best results.

### Example 5: EGFP expression levels after mRNA transfection with defined double-modified PPI-G3 or -G4 derivatives

Beyond the total fluorescence signal determined above *(i.e.,* total integrated fluorescence intensity), EGFP expression levels in the transfected cells as represented by the fluorescence intensities are of interest as well. Here, PPI-G4-Y(60/40)/mRNA transfected cells showed largely similar EGFP levels as compared to their PPI-G4-Y(0/100) or JM-transfected counterparts **(****Fig. 1B****).** Bearing in mind the higher total integrated fluorescence intensity compared to PPI-G4-Y(0/100) or JM shown in **Fig. 1A****,** this indicates that a larger number of cells are EGFP positive after PPI-G4(60/40)/mRNA transfection, while the EGFP expression levels of the positive cells are comparable. The variation of transfected mRNA amounts (0.25, 0.5, 1.0 µg/well) revealed essentially no dose-dependency, with JM slightly benefitting from larger mRNA amounts. In the case of PPI-G4-Y(30/70), expression levels were even below JM **(****Fig. 2B****),** despite higher total integrated fluorescence intensities **(****Fig. 2A****).** Again, this demonstrates that a larger number of cells were EGFP positive upon PPI-G4-Y(30/70)/mRNA transfection.

Notably, when employing PPI-G3-Y(60/0) for transfection, substantially higher EGFP expression levels were achieved over JM or the Tyr-monosubstituted PPI-G3-Y(0/100) **(****Fig. 3B****).** Especially at the optimal mRNA amount (0.5 µg/well), transfection efficacies benefitted from the higher polymer/mRNA mass ratios (3.75, 5.0) as compared to ratio 2.5.

Similarly, mRNA complexes based on the double-modified PPI-G3(60/40) achieved very high EGFP expression, with even slightly better results at mass ratios 3.75 and 5.0 **(****Fig. 4B****).** No mRNA dose-dependence was observed, while high EGFP levels even upon transfection of larger mRNA amounts PPI-G3-Y(60/40)-complexed at the highest tested ratio 5.0 also indicated the absence of cytotoxic effects.

### Example 6: Double-modified PPI-G3/-G4 derivatives for self-amplifying mRNA (sa-mRNA) transfection

For achieving long-term expression, a self-amplifying mRNA (sa-mRNA) was employed, encoding a viral replicase in addition to the gene to be expressed. The replicase can amplify the sa-mRNA in transfected cells, potentially reducing the amount of required RNA while increasing the duration of transgene expression. A serious limitation, however, lies in the fact that gene constructs encoding sa-mRNAs, as well as the sa-mRNA itself, are very large due to the requirement for encoding additional proteins. While this may easily become prohibitive for using viral vectors, it may also impose high requirements on non-viral delivery systems.

The present inventors next analyzed the efficacy of different PPI-Y derivatives for the transfection of self-amplifying mRNA (sa-mRNA), compared to JM. The transfection efficacies, determined in the Incucyte instrument by measuring collective EGFP expression in the well (total green object area), revealed substantially higher values over JM in the case of PPI-G4-Y(60/0)/mRNA complexes and PPI-G3-Y(60/40)/mRNA complexes **(****Fig. 5A****).** Complexes were prepared at the low polymer/sa-mRNA mass ratio 2.5 and contained 0.5 µg sa-mRNA. The curves also indicate a slower (compared to mRNA; see **Fig. 4A**) onset of EGFP expression after ~ 12 h, with an upper plateau being reached after ~ 2 days **(****Fig. 5A****).** Importantly, no subsequent decay in EGFP expression was seen even when cells were cultivated over several weeks. While non-transfected, EGFP-negative cells rapidly grew as well, confocal laser scanning microscopy of the cell cultures after 1.5 months (day 48 after transfection) still revealed the presence of EGFP positive cells **(****Fig. 5B****).** More specifically, the repeated trypsinization and re-seeding of the cells at lower densities ('cell splitting') that is required for continuous cultivation over longer time periods in order to avoid over-confluency and medium exhaustion revealed that positive (and negative) cells formed individual colonies due to cell division (**Fig. 5B****;** compare fluorescence (left) with bright field (right)). Notably, all daughter cells in the colony derived from a single EGFP-positive cell were found to be equally positive, indicating the absence of mRNA dilution in the process of cell division and constantly high mRNA levels available for translation. Thus, this demonstrates that the herein provided PPIs lead to the successful transfection of a cell with the sa-mRNA, that leads to persistent expression of EGFP also in all its daughter cells after cell division, as indicated by the colony of 100% EGFP positive cells.

### Example 7: Efficacy of double-modified PPI-G3/-G4 derivatives for DNA transfection

Beyond mRNA transfection, transient ectopic overexpression can also be achieved by transfecting DNA. This approach may employ standard size DNA expression plasmids as well as smaller, so-called minicircle DNA plasmid devoid of sequences not required for transcription and gene expression. Both DNA vectors were used for the assessment of DNA transfection efficacies of PPI-G3-Y derivatives PPI-G3-Y(60/0) and PPI-G3-Y(60/40). All transfections led to EGFP positivity of the cells. EGFP expression was higher upon transfection of minicircle DNA as compared the larger plasmid DNA. In the case of both DNA vectors, higher efficacies were observed when using the double-modified PPI-G3-Y(60/40) for complexation. Thus, the PPI-G3-Y(60/40)/minicircle-DNA transfection yielded particularly high ectopic EGFP expression **(****Fig. 6****).**

Chemically double-modified, 4^{th} generation PPI-G4-based dendrimers displayed DNA transfection efficacy as well. Notably, when comparing PPI-G4-Y(60/40) and PPI-G4-Y(30/70), the latter displayed even higher transfection efficacies. This was true with regard to the number of EGFP-positive cells **(****Fig. 7A****)** as well as their EGFP expression levels, as determined by mean fluorescence intensities **(****Fig. 7B****).**

### Example 8: Efficacy of double-modified PPI-G3/-G4 derivatives for inducing siRNA-mediated gene knockdown

Beyond gain-of-function transfections relying on mRNA, the transfection of very small RNAs like *e.g.* siRNAs for the induction of RNAi-mediated gene knockdown is of major interest as well. These very small and rather rigid RNA molecules, however, impose other requirements on the delivery reagent. Thus, the present inventors next studied the efficacies of the different single- or double-modified PPI-G3/-G4 dendrimers for the formulation and delivery of siRNA into stably luciferase-expressing PC3-Luc reporter cells. PPI-G4-Y(0/100) showed ~ 80% knockdown efficacy, as indicated by the difference between luciferase activities (RLU) of cells transfected with negative control siRNA (siCtrl, black bars; normalized to 100%) and luciferase-specific siRNA (siLuc, grey bars) **(****Fig. 8A****).** Here and elsewhere, no differences were seen between different polymer/siRNA ratios, *i.e.,* ratio 2.5, 3.75 and 5.0. Notably, the dendrimers PPI-G4-Y(100/0) with single BOC-Tyr modification did not lead to gene knockdown (presumably also due to precipitation issues), while PPI-G4 with lesser degrees of BOC-Tyr mono-substitution (PPI-G4-Y(60/0) and PPI-G4-Y(30/0)) did show knockdown activity. In contrast, and PPI-G3-Y(60/0) revealed no knockdown activity. In particular the assessment of the different PPI-Y derivatives with combined BOC-tyrosine and tyrosine modification, however, led to high luciferase knockdown by siRNA-mediated induction of RNAi. This was true for all double-modified PPI-G3- and PPI-G4 derivatives with BOC-Tyr modification degrees exceeding the Tyr modification, *i.e.,* a 60/40 ratio **(****Fig. 8A****).**

Similar results were observed in H441-Luc reporter cells **(****Fig. 8B****).** Here, however, high knockdown efficacies were also found for PPI-G4-Y(30/70). In all experiments, essentially no dependence on the polymer/siRNA mass ratio was observed, with a ratio 2.5 already being sufficient for achieving maximum knockdown efficacies. These were well above 90% in most cases.

### Example 9: PPI-G3-Y(60/40) for sgRNA delivery and induction of base editing

Base editing represents a rather novel application of RNAs, leading to the targeted mutation of a target DNA. Pivotal is the cellular internalization and intracellular release of the specific RNAs (called single guide RNAs, sgRNA) required for base editing. Based on the above results on mRNA delivery, the present inventors selected PPI-G3-Y(60/40) for further studies. To analyze efficacies of PPI-G3-Y(60/40)-based nanoparticles for delivering RNAs for the induction of base editing, the present inventors employed a PC3 green-fluorescent reporter cell line and an sgRNA +Cas-mRNA complex inducing a point mutation. This base editing event leads to a shift of the emission wavelength of EGFP towards blue fluorescence. In flow cytometry, this was indicated by the shift of cells from the upper left to the lower right quadrant.

Indeed, the flow cytometry histograms of cells prior and after PPI-G3-Y-based introduction of the base editing RNAs revealed a decrease of cell percentages in the upper left and increase of cell percentages in the lower right quadrant, respectively **(****Fig. 9A****).** A similar experiment performed with complexes prepared at a higher polymer/RNA mass ratio (3.75 instead of 2.5) led to even further increased efficacies. Thus, this example introduces double-modified PPI-derivatives for the delivery of RNAs inducing base editing. This shift in emission wavelength was seen by an increase in blue fluorescence as well as a decrease in green fluorescence **(****Fig. 9B****).**

### Example 10: Use of PPI-G3-Y(60/40) and PPI-G4-Y(60/40) for in vivo mRNA delivery

Beyond the use of nanoparticles as transfection reagent for *in vitro* application, the delivery of RNA *in vivo* is of particular interest. Thus, the efficacy of PPI-G3-Y(60/40)- or PPI-G4-Y(60/40)-based nanoparticles for mRNA delivery *in vivo* was studied. Mice were intraperitoneally injected with 20 µg or 40 µg luciferase-encoding mRNA, respectively, formulated with PPI-G3-Y(60/40) or PPI-G4-Y(60/40). 6 h or 24 h after injection of the nanoparticles, luciferase expression was determined *via* live imaging of the anesthetized animals. Profound expression in particular in the abdominal cavity was observed for both assayed nanoparticles, indicating nanoparticle-mediated delivery of intact mRNA and its translation into the encoded protein (EGFP). In the case of both double-modified dendrimers, this was seen as early as 6 h after injection (**Fig. 10A** and **Fig. 10B****,** left panels) and persisted over at least 24 h (right panels).

### Example 11: Efficacy of BOC-modified PEI derivatives for DNA transfection

Given the above-described, surprising and highly encouraging results obtained for single and double modified PPIs, the present inventors aimed at assessing whether BOC-modified polyethylenimines (PEIs) would also show advantages over tyrosine modified PEIs. To this end, the present inventors generated , double-modified polymers *(i.e.,* comprising both BOC- and tyrosine-modifications; see Example 1.B). The **Formulas III** and **IV** provide exemplary chemical structures of a branched and a linear PEI, respectively, with R representing the tyrosine modification or the BOC-tyrosine modification in a ratio defined by percentages. The branched PEI (*e.g.,* P10Y) is primarily modified at primary amines which account for approx. 30% of all amines. In the case of the linear PEI (*e.g.,* LP10Y), approximately 35% of all amines are modified. The syntax describing these modifications is as follows: P10Y(yy)(xxᵣₑₗ/zzᵣₑₗ) or LP10Y(yy)(xxᵣₑₗ/zzᵣₑₗ); with yy = percent degree of total modification (in particular amine modification), and related to these modified amines xxᵣₑₗ = relative percentage of BOC-tyrosine modification and zzᵣₑₗ = relative percentage of tyrosine modification. For example, LP10Y(35)(50/50) describes a 10 kDa linear PEI with a 35% total degree of modification of all amines. 50% of those modified amines bear BOC-tyrosine and 50% bear tyrosine. These relative percentages/percent degrees of BOC- and tyrosine-modifications can readily be computed into the total/absolute BOC- and tyrosine-modification. For example the linear PEI LP10Y(35)(50/50), comprises in total about 18% BOC-modifications (*i.e.,* 50%/100% x 30% = 18%) and in total about 18% tyrosine modifications (*i.e.,* 50%/100% x 30% = 18%). Such calculations can readily be applied to determine the total/absolute percentages/percent degree of BOC-, tyrosine-, and Fmoc-modification of the herein provided linear and branched PEI polymers (when the relative BOC- and tyrosine percentages/percent degrees are indicated in the '(yy)(xxᵣₑₗ/zzᵣₑₗ)'-format). Accordingly, such a calculation would read: 'xxᵣₑₗ/100 x yy = xx' to determine the total percentages of BOC-modifications or 'zzᵣₑₗ/100 x yy = zz' to determine the total/absolute percentages of tyrosine-modifications. In this regard, the following also holds true: yy = xx + zz.

The analysis of plasmid DNA transfection efficacies of the different P10Y derivatives revealed that the introduction of in total 3% or 15% of the BOC-Tyr modification (*i.e.,* when employing P10Y(30)(10/90) and P10Y(30)(50/50), respectively) led to an increase of the percentage of EGFP positive cells from ~ 20 % (Tyr modification) to ~ 80 % (double modification with in total 103 BOC-Tyr or 15% BOC-Tyr; **Fig. 11A**). Additionally, the quantitation of the mean fluorescence intensities of EGFP-positive cells revealed a substantial, almost 10-fold increase in fluorescence intensity, *i.e.,* in the EGFP expression levels of transfected cells **(****Fig. 11B****).** Thus, substantially improved DNA transfection efficacies of the double-modified PEIs were observed over their single tyrosine-modified, branched 10 kDa counterpart (P10Y) that was established previously [2] and thus represents the state of the art.

### Example 12: Efficacy of double-modified PEI derivatives for inducing siRNA-mediated gene knockdown

As described above, the transfection of very small oligonucleotides, *e.g.*, siRNAs for RNAi-mediated gene knockdown, is another field of major interest, but may impose other requirements on the delivery reagent. Thus, the efficacies of different P10Y derivatives for the transfection of siRNA into stably luciferase-expressing H441/Luc reporter cells were analyzed. Compared with the tyrosine-modified branched PEI of the state of the art P10Y published previously [2] and being state of the art, the novel branched PEIs with combined BOC-tyrosine and tyrosine modification show enhanced luciferase knockdown upon siRNA-mediated transfection, leading to the induction of RNAi, as indicated by the difference between luciferase activities (RLU) of cells transfected with negative control siRNA (siCtrl, black bars; normalized to 100%) or luciferase-specific siRNA (siLuc, grey bars). The polymer particularly benefitted from a degree BOC-Tyr modification at 7.5% or higher (*i.e.,* P10Y(30)(25/75), and P10Y(30)(50/50); **Fig. 12**). A very low polymer/siRNA mass ratio 2.5 was already sufficient for maximum knockdown efficacy well above 90%, which was not achieved with P10Y (*i.e.,* the state of the art branched PEI that is only modified with tyrosine).

## Claims

1. A composition comprising a polypropylenimine-dendrimer (PPI) or a polyethylenimine (PEI), wherein said PPI or said PEI comprises one or more N-Boc-tyrosine residue(s), wherein the composition further comprises one or more agent(s), wherein said PEI is a linear PEI or a branched PEI, and wherein said one or more agent(s) is/are one or more polynucleotide molecule(s), one or more polypeptide(s), and/or one or more small molecule(s), preferably one or more polynucleotide molecule(s).

2. The composition according to claim 1, wherein said PPI or said PEI forms a nanoparticle and/or is formulated as a nanoparticle with said one or more agent(s).

3. The composition according to claim 1 or 2, wherein said one or more agent(s) are one or more polynucleotide molecule(s) selected from the group consisting of DNA, RNA, mRNA, self-amplifying mRNA (sa-mRNA), minicircle DNA, DNA plasmid, small inhibitory RNA (siRNA), single guide RNA (sgRNA), microRNA (miRNA), anti-miRNA (antimir), Gapmer, and circular RNA (circRNA).

4. A polypropylenimine-dendrimer (PPI) or a polyethylenimine (PEI), wherein said PPI or said PEI comprises one or more N-Boc-tyrosine residue(s), and wherein said PEI is a linear PEI or a branched PEI.

5. The composition according to any one of claims 1 to 3, or the PPI or the PEI according to claim 4, wherein between about 20% and about 80%, preferably between about 40% and about 70% of all terminal amino groups of said PPI are linked to an N-Boc-tyrosine residue, wherein between about 1% and about 64%, preferably between about 3% and about 15% of all terminal amino groups of said branched PEI are linked to an N-Boc-tyrosine residue and/or wherein between about 1% and about 60%, preferably between about 10% and about 20% of all nitrogen groups of said linear PEI are linked to an N-Boc-tyrosine residue.

6. The composition according to any one of claims 1 to 3 and 5, or the PPI or the PEI according to claim 4 or 5, wherein said PPI or said PEI further comprises one or more tyrosine residue(s), preferably wherein said one or more tyrosine residue(s) are each linked to one or more terminal amino group(s) of said PPI or to one or more amino group(s) of said branched PEI or to one or more nitrogen group(s) of said linear PEI.

7. The composition according to any one of claims 1 to 3, 5 and 6, or the PPI or the PEI according to any one of claim 4 to 6, wherein between about 0% and about 80%, preferably between about 30% and about 60% of all terminal amino groups of said PPI are linked to a tyrosine residue, wherein between about 2% and about 57% of all terminal amino groups of said branched PEI are linked to a tyrosine residue, and wherein between about 0% and about 57% of all nitrogen groups of said linear PEI are linked to a tyrosine residue.

8. The composition according to any one of claims 1 to 3, and 5 to 7, or the PPI according to any one of claims 4 to 7, wherein said PPI is a PPI of the second, third, or fourth generation, preferably of the third or the fourth generation.

9. The composition according to any one of claims 1 to 3, and 5 to 8, or the PPI according to any one of claims 4 to 8, wherein about 60% of all terminal amino groups of said PPI are linked to an N-Boc-tyrosine residue, and wherein about 40% of all terminal amino groups of said PPI are linked to a tyrosine residue.

10. The composition according to any one of claims 1 to 3, and 5 to 7, or the PEI according to any one of claims 4 to 7, wherein said PEI has a molecular weight between about 2 kDa and about 25 kDa, preferably between about 5 kDa and about 15 kDa.

11. The composition according to any one of claims 1 to 3, and 5 to 10 for use in the treatment, the prevention, and/or the diagnosis of a disease, wherein said one or more agent(s) are one or more therapeutic agent(s), and/or one or more diagnostic agent(s).

12. A method for the transfection of a cell *in vitro* or *ex vivo,* the method comprising contacting said cell with the composition according to any one of claims 1 to 3 and 5 to 10.

13. A method for the manufacture of a composition according to any one of claims 1 to 3 and 5 to 10, for the manufacture of a PPI according to any one of claims 4 to 9 or for the manufacture of a PEI according to any one of claims 4 to 7 and 10.

14. The method according to claim 13, wherein the to be produced PPI is a PPI comprising one or more N-Boc-tyrosine residue(s) and one or more tyrosine residue(s) or wherein the to be produced PEI is a PEI comprising one or more N-Boc-tyrosine residue(s) and one or more tyrosine residue(s), wherein the method comprises the steps of:
(i) reacting a PPI or a PEI with N-Boc-tyrosine and with a further N-terminally protected-tyrosine residue;
(ii) deprotecting said further N-terminally protected-tyrosine residue,
preferably wherein said further N-terminally protected-tyrosine residue is a tyrosine residue N-terminally protected by a N-terminal protection group selected from the group consisting of Fmoc, Smoc (2,7-disulfo-9-fluorenylmethoxycarbonyl), TFA (Trifluoromethylacetyl), Dde (1-(4,4-dimethyl-2,6-dioxocyclohex-1-ylidene)ethyl), Teoc (2-(trimethylsilyl)ethoxycarbonyl), pNB (para-Nitrobenzyl), and Nosyl (Nitrobenzensulfonyl), more preferably Fmoc.

15. A kit comprising:
(i) a PPI according to any one of claims 4 to 9 or a PEI according to any one of claims 4 to 7 and 10; and
(ii) a buffer.
